# EUROPÄISCHE PATENTSCHRIFT

(11) **EP 1 877 518 B1**
(45) Veröffentlichungstag und Bekanntmachung des Hinweises auf die Patenterteilung: **24.02.2010**
(21) Anmeldenummer: 06743236.9
(22) Anmeldetag: 03.04.2006
(51) Int. Cl.: C09K 11/06, H05B 33/14, C08G 61/10, C07C 229/74, C07C 13/68

(54) **SYNTHESE VON PHENYLSUBSTITUIERTEN POLYFLUORANTHENEN UND IHRE VERWENDUNG**
SYNTHESIS OF PHENYL-SUBSTITUTED POLYFLUOROANTHENES AND THE USE THEREOF
SYNTHESE DE POLYFLUOROANTHENES A SUBSTITUTION PHENYLE ET UTILISATION

(30) Priorität: 28.04.2005 DE 102005019880
(43) Veröffentlichungstag der Anmeldung: 16.01.2008
(73) Patentinhaber: BASF SE, 67056 Ludwigshafen (DE)
(72) Erfinder: DÖTZ, Florian, 69120 Heidelberg (DE); ROESCH, Joachim, 67063 Ludwigshafen (DE); WEISS, Horst, 67141 Neuhofen (DE); NORD, Simon, 67354 Römerberg (DE)
(86) Internationale Anmeldenummer: PCT/EP2006/061282
(87) Internationale Veröffentlichungsnummer: WO 2006/114364

(56) Entgegenhaltungen:
- WO-A-2004/095888
- WO-A-2005/033051
- DE-A1- 10 211 648
- GHOSH K ET AL: "SYNTHESIS OF ARYLATED FLUORANTHENE DERIVATIVES" INDIAN JOURNAL OF CHEMISTRY, JODHPUR, IN, Bd. 16B, Nr. 2, 1. Februar 1978 (1978-02-01), Seiten 152-153, XP000570586

## Beschreibung

Die vorliegende Erfindung betrifft ein Verfahren zur Herstellung von Polyfluoranthenderivaten, Polyfluoranthenderivate herstellbar nach dem erfindungsgemäßen Verfahren, Filme enthaltend oder bestehend aus mindestens einem erfindungsgemäßen Polyfluoranthenderivat, organische Leuchtdioden (OLEDs) enthaltend mindestens ein erfindungsgemäßes Polyfluoranthenderivat, eine Licht-emittierende Schicht enthaltend oder bestehend aus mindestens einem erfindungsgemäßen Polyfluoranthenderivat, ein O-LED enthaltend die erfindungsgemäße Licht-emittierende Schicht, Vorrichtungen, enthaltend ein erfindungsgemäßes OLED, sowie die Verwendung der erfindungsgemäßen Polyfluoranthenderivate als Emittersubstanzen in OLEDs.

In organischen Leuchtdioden (OLEDs) wird die Eigenschaft von Materialien ausgenutzt, Licht zu emittieren, wenn sie durch elektrischen Strom angeregt werden. OLEDs sind insbesondere interessant als Alternative zu Kathodenstrahlröhren und Flüssigkristalldisplays zur Herstellung von Flachbildschirmen.

Es wurden zahlreiche Materialien vorgeschlagen, die bei Anregung durch elektrischen Strom Licht emittieren.

Eine Übersicht über OLEDs ist zum Beispiel in M. T. Bernius et al., Adv. Mat. 2000, 12, 1737 offenbart. Die Anforderungen an die verwendeten Verbindungen sind dabei hoch und es gelingt mit den bekannten Materialien üblicherweise nicht, alle gestellten Anforderungen zu erfüllen.

Neben anorganischen und niedermolekularen organischen Elektrolumineszenzmaterialien wird im Stand der Technik auch der Einsatz von polymeren Elektrolumineszenzmaterialien in OLEDs beschrieben. Die Aufbringung von anorganischen und niedermolekularen organischen Elektrolumineszenzmaterialien als Licht-emittierende Schicht in OLEDs erfolgt im Allgemeinen durch Dampfabscheidung des anorganischen oder niedermolekularen organischen Materials im Vakuum. Ein Dampfabscheidungsverfahren ist jedoch zur Massenproduktion von OLEDs ungeeignet und unterliegt Einschränkungen im Hinblick auf die Herstellung von Vorrichtungen mit großflächigen Displays. Des Weiteren ist es problematisch, aus niedermolekularen organischen Elektrolumineszenzmaterialien durch Beschichtung einen dünnen Film herzustellen, wenn diese Materialien ohne Matrixmaterialien verwendet werden.

Demgegenüber haben polymere Elektrolumineszenzmaterialien den Vorteil, dass sie in Form eines Films aus Lösung aufgebracht werden können, beispielsweise durch Spincoating oder Tauchen, was es ermöglicht, großflächige Displays einfach und kostengünstig herzustellen.

WO 90/13148 betrifft OLEDs, die Polymere auf der Basis von Poly(p-phenylen-vinylen) (PPV) enthalten. Derartige Polymere sind besonders für die Elektrolumineszenz im roten und grünen Spektralbereich geeignet.

Im blauen Spektralbereich werden meist Derivate des Poly(fluoren)s (PF) verwendet. Poly(fluoren)derivate, die Spirozentren aufweisen, sind zum Beispiel in EP-A 0 707 020 offenbart.

Die vorstehend erwähnten PPV- und PF-Derivate verfügen zwar meist über zufrieden stellende optische Eigenschaften wie Emissionsfarbe und Quantenausbeute der Emission, es mangelt Ihnen jedoch im Allgemeinen an der erforderlichen Langzeitstabilität. Die Gründe hierfür reichen von morphologischer Instabilität über Excimerbildung bis zu oxidativem Abbau des Chromophors.

Polymere und Copolymere enthaltend Wiederholungseinheiten auf Basis von Fluoranthen und dessen Derivaten sind aus dem Stand der Technik bekannt.

R. J. Waltmann et al., J. Electrochem. Soc. 1985, 132, 631 bis 634 betrifft Polymere, die durch die Elektropolymerisation von Fluroanthen erhalten werden. Es werden strukturell sehr uneinheitliche Polymere erhalten, deren schlechte Löslichkeit und Prozessierbarkeit eine eingehende Charakterisierung verhindert. Filme aus den durch Elektropolymerisation von Fluoranthen erhaltenen Polymeren sind brüchig und weisen eine geringe Leitfähigkeit auf.

L. Dunsch et al., Angew. Chem. 2002, 114, 12, 2187 bis 2190 betrifft Fluoranthenopyrazylen-Oligomere, die durch repetitive Diels-Alder-Reaktion und anschließende Aromatisierung erhalten werden. Als Diene werden Cyclopentadienonderivate eingesetzt, die unter cheleotroper Kohlenmonoxideleminierung mit geeigneten Dienophilen reagieren.

In J. K. Stille et al., J. Polym. Sci. Part A 1970, 8, 2245 bis 2254 ist die Herstellung von aromatischen Leiterpolymeren durch Diels-Alder-Homopolycycloaddition beschrieben. Als Monomere werden Cyclopentadienonderivate eingesetzt, die gleichzeitig eine Dien- und eine Dienophil-Einheit in demselben Molekül aufweisen. Diese Monomere reagieren durch Diels-Alder-Polycycloaddition unter Kohlenmonoxid-Eliminierung zu den gewünschten Leiterpolymeren.

R. A. Gaudiana et al., Macromolecules 1995, 28, 368 bis 389 betrifft Polymethacrylate und Polysiloxane, die lumineszierende Seitengruppen aufweisen. Beispielsweise ist ein Polymethacrylat mit Fluoranthenseitengruppen, Poly[2-(3-fluoranthenylureido)ethylmethacrylat], offenbart. Diese Polymere können in OLEDs eingesetzt werden.

DE-A 102 11 648 betrifft Polymere auf Basis von Fluoranthen und ihre Verwendung in OLEDs. Zur Herstellung dieser Polymere werden funktionalisierte Fluoranthene polymerisiert. Die Funktionalisierung der Fluoranthene gemäß DE-A 102 11 648 erfolgt durch Halogenierung von Fluoranthen oder durch Nitrierung von Fluoranthen und entsprechende weitere Umsetzung zu den gewünschten Monomeren.

WO 2005/033051 betrifft die Synthese von phenylsubstituierten Fluoranthenderivaten durch Diels-Alder-Reaktion. Die Fluoranthenderivate gemäß WO 2005/033051 werden nicht durch Polymerisation hergestellt. Es handelt sich somit nicht um Polymere (mit einer Molekulargewichtsverteilung), sondern um definierte Verbindungen, die sich strukturell deutlich von durch Polymerisation hergestellten Polyfluoranthenderivaten unterscheiden und andere physikalische Eigenschaften aufweisen als durch Polymerisation hergestellte Polyfluoranthenderivate.

WO 2004/095888 betrifft eine OLED, die eine organische Schicht aufweist, die ein Vinylpolymer enthält. Das Vinylpolymer ist durch Polymerisation von polymerisierbaren Fluoranthenderivaten erhältlich. Die polymerisierbaren Fluoranthenderivate weisen entweder in 1-, 2- oder 3-Position oder in 7-, 8-, 9- oder 10-Position eine olefinische Doppelbindung, bevorzugt eine vinylische Doppelbindung auf. Bei einer Polymerisation der Fluoranthenmonomere werden Vinylpolymere enthalten, die Fluoranthen-Seitenketten enthalten. Die Polymere gemäß WO 2004/095888 weisen somit kein konjugiertes π-System auf, sondern sind Vinylpolymere, die frei rotierbare Fluoranthen-Seitenketten aufweisen.

Aufgabe der vorliegenden Erfindung ist es weitere Polyfluoranthenderivate bereitzustellen, die zum Einsatz in OLEDs, insbesondere als Emittermoleküle, geeignet sind, eine lange Lebensdauer aufweisen, in OLEDs hocheffizient sind, ein Emissionsmaximum im blauen Bereich und eine hohe Quantenausbeute aufweisen. Des Weiteren ist es Aufgabe der vorliegenden Erfindung, ein Verfahren zur Herstellung solcher Polyfluoranthene bereit zu stellen.

Diese Aufgabe wird gelöst durch ein Verfahren zur Herstellung von Polymeren enthaltend Wiederholungseinheiten der allgemeinen Formel I 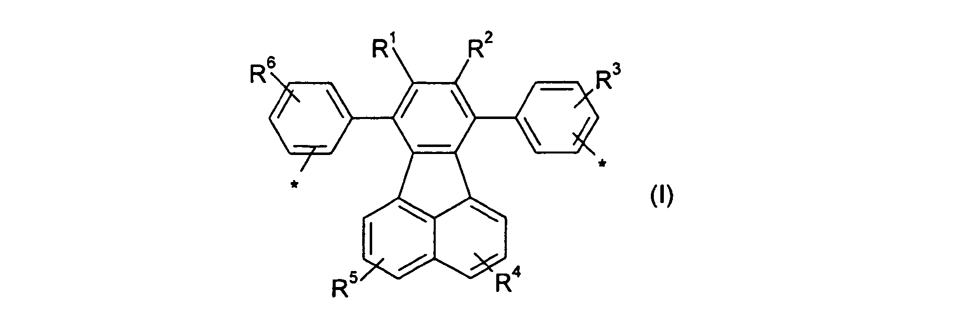 umfassend folgende Schritte:
a) Herstellung eines monomeren Fluoranthenderivats der Formel IIa 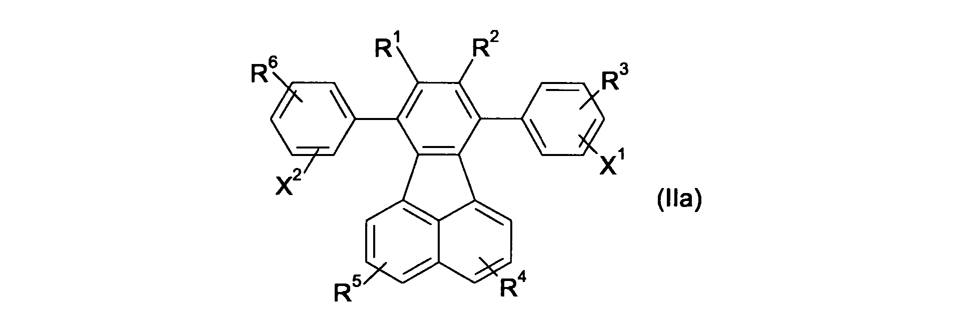 durch Reaktion einer Verbindung der Formel III 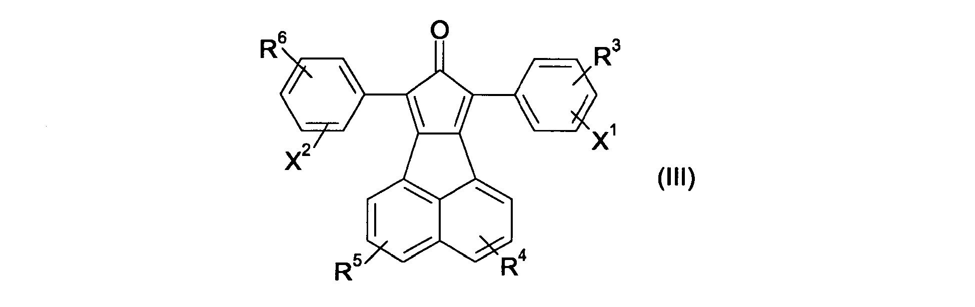 mit einer Alkinylverbindung der Formel IV 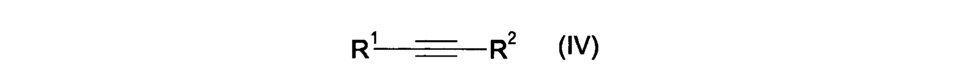 und anschließender Kohlenmonoxid-Eliminierung;
b) gegebenenfalls Umsetzung des monomeren Fluoranthenderivats der Formel IIa zu einem monomeren Fluoranthenderivat der Formel IIb 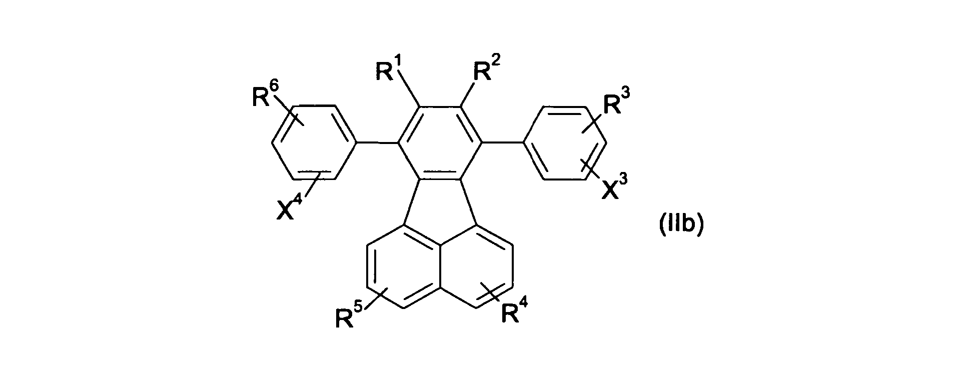
c) Polymerisation des monomeren Fluoranthenderivats der Formel IIa oder IIb, gegebenenfalls gemeinsam mit mindestens einem weiteren Comonomeren ausgewählt aus der Gruppe bestehend aus einem weiteren von dem ersten Fluoranthenderivat der Formel IIa oder IIb verschiedenen Fluoranthenderivat der Formel IIa oder IIb, aromatischen, kondensierten aromatischen und heteroaromatischen Verbindungen, die jeweils zwei mit den Gruppen X¹ und X² des Fluoranthenderivats der Formel IIa bzw. den Gruppen X³ und X⁴ des Fluoranthenderivats der Formel IIb polymerisierbare Gruppen X⁵ und X⁶ aufweisen;
   wobei die Symbole die folgenden Bedeutungen aufweisen:
   - R¹, R², R³ R⁴, R⁵, R⁶: unabhängig voneinander H, ein Alkylrest, ein Alkinylrest, ein Aryloxy- rest, ein aromatischer Rest, ein kondensiertes aromatisches Ringsys- tem, ein heteroaromatischer Rest, -CH=CH₂, (E)- oder (Z)-CH=CH- C₆H₅, Acryloyl, Methacryloyl, Methylstyryl, -O-CH=CH₂ oder Glycidyl, 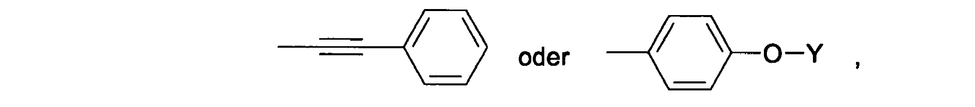 bedeuten,
   wobei Y Acryloyl, Methacryloyl, ortho- oder para-Methylstyryl, -O- CH=CH₂ oder Glycidyl bedeutet;
   - X¹, X²,X³, X⁵, X⁶: X⁴ miteinander polymerisierbare Gruppen.

Derart ausgedehnte, Phenyl-substituierte Fluoranthenderivate gemäß Formel IIa oder IIb sind in definierter, strukturtreuer Weise auf herkömmlichem Wege nicht mit polymerisationsfähigen Gruppen X¹ und X² bzw. X³ und X⁴ zu versehen. Beispielsweise ergibt die Bromierung von 7,8,9,10-Tetraphenylfluoranthen Isomerenmischungen und die selektive Einführung von Bromäquivalenten gelingt nicht. In Schritt a) des erfindungsgemäßen Verfahrens werden die polymerisierbaren Gruppen X¹ und X² daher durch Neuaufbau des Fluoranthengerüsts, wobei die polymerisierbaren Gruppen bereits auf der ersten Stufe der Synthese eingefügt werden, selektiv in das Phenyl-substituierte Fluoranthen eingeführt. Schlüsselreaktion ist dabei die Diels-Alder-Reaktion des Diencyclopentaacenaphthenon, das mit den polymerisierbaren Gruppen X¹ und X² substituiert ist, mit einem Acetylenderivat als Dienophil unter cheleotroper Eliminierung von Kohlenmonoxid.

Im Sinne der vorliegenden Anmeldung bedeutet "Alkyl" eine lineare, verzweigte oder cyclische substituierte oder unsubstituierte C₁- bis C₂₀-, bevorzugt C₁- bis C₁₀-Alkylgruppe. Besonders bevorzugt handelt es sich um eine lineare oder verzweigte C₃-bis C₁₀-, ganz besonders bevorzugt C₅- bis C₉- Alkylgruppe. Die Alkylgruppen können unsubstituiert oder mit aromatischen Resten, Halogen-, Nitro-, Ether- oder Carboxylgruppen substituiert sein. Besonders bevorzugt sind die Alkylgruppen unsubstituiert oder mit aromatischen Resten substituiert. Bevorzugte aromatische Reste sind nachstehend genannt. Des Weiteren können ein oder mehrere nicht benachbarte Kohlenstoffatome der Alkylgruppe durch Si, P, O oder S, bevorzugt durch O oder S, ersetzt sein. Unter Halogengruppen sind bevorzugt F, Cl oder Br zu verstehen.

Im Sinne der vorliegenden Anmeldung bedeutet "Alkinyl" eine lineare, verzweigte oder cyclische substituierte oder unsubstituierte C₂- bis C₂₀-, bevorzugt C₂- bis C₁₀-Alkinylgruppe. Besonders bevorzugt handelt es sich um eine lineare oder verzweigte C₂- bis C₈-, ganz besonders bevorzugt C₂- bis C₆- Alkinylgruppe. Die Alkinylgruppen können unsubstituiert oder mit aromatischen Resten, Halogen-, Nitro-, Ether- oder Carboxylgruppen substituiert sein. Besonders bevorzugt sind die Alkinylgruppen unsubstituiert oder mit aromatischen Resten substituiert. Bevorzugte aromatische Reste sind nachstehend genannt.

Im Sinne der vorliegenden Anmeldung bedeutet Aryloxy eine -O-Ar-Gruppe. Die Arylgruppe in dem Aryloxyrest ist bevorzugt eine C₆-Arylgruppe (Phenylgruppe) oder Naphthylgruppe, besonders bevorzugt eine Phenylgruppe. Diese Arylgruppe kann unsubstituiert oder mit linearen, verzweigten oder cyclischen C₁- bis C₁₀-, bevorzugt C₁- bis C₉-Alkylgruppen substituiert sein, die wiederum mit Halogen-, Nitro-, Ether- oder Carboxylgruppen substituiert sein können. Des Weiteren können ein oder mehrere Kohlenstoffatome der Alkylgruppe durch Si, P, O, S oder N, bevorzugt O oder S, ersetzt sein. Des Weiteren können die Arylgruppen mit Halogen-, Nitro-, Carboxylgruppen, Aminogruppen oder Alkoxygruppen oder C₆- bis C₁₄-, bevorzugt C₆- bis C₁₀-Arylgruppen, insbesondere Phenyl- oder Naphthylgruppen, substituiert sein. Unter Halogengrupen sind bevorzugt F, Cl oder Br zu verstehen. Besonders bevorzugt ist die Arylgruppe in dem Aryloxyrest eine C₆-Arylgruppe, die gegebenenfalls mit Halogen, bevorzugt Br, Cl oder F, Aminogruppen, bevorzugt NAr'Ar", wobei Ar' und Ar" unabhängig voneinander C₆-Arylgruppen bedeuten, die, wie vorstehend definiert, unsubstituiert oder substituiert sein können und/oder Nitrogruppen substituiert ist. Ganz besonders bevorzugt ist diese Arylgruppe in dem Aryloxyrest unsubstituiert.

Im Sinne der vorliegenden Anmeldung bedeutet "aromatischer Rest" im allgemeinen eine Arylgruppe, bevorzugt eine C₆-Arylgruppe (Phenylgruppe) oder Naphthylgruppe, besonders bevorzugt eine Phenylgruppe. Diese Arylgruppe kann unsubstituiert oder mit linearen, verzweigten oder cyclischen C₁- bis C₁₀-, bevorzugt C₁- bis C₉-Alkylgruppen substituiert sein, die wiederum mit Halogen-, Nitro-, Ether- oder Carboxylgruppen substituiert sein können. Des Weiteren können ein oder mehrere Kohlenstoffatome der Alkylgruppe durch Si, P, O, S oder N, bevorzugt O oder S, ersetzt sein. Des Weiteren können die Arylgruppen mit Halogen-, Nitro-, Carboxylgruppen, Aminogruppen oder Alkoxygruppen oder C₆- bis C₁₄-, bevorzugt C₆- bis C₁₀-Arylgruppen, insbesondere Phenyl- oder Naphthylgruppen, substituiert sein. Unter Halogengrupen sind bevorzugt F, Cl oder Br zu verstehen. Besonders bevorzugt bedeutet "aromatischer Rest" eine C₆-Arylgruppe, die gegebenenfalls mit Halogen, bevorzugt Br, Cl oder F, Aminogruppen, bevorzugt NAr'Ar", wobei Ar' und Ar" unabhängig voneinander C₆-Arylgruppen bedeuten, die, wie vorstehend definiert, unsubstituiert oder substituiert sein können und/oder Nitrogruppen substituiert ist. Ganz besonders bevorzugt ist diese Arylgruppe unsubstituiert oder mit NAr'Ar" substituiert.

Im Sinne der vorliegenden Anmeldung bedeutet "kondensiertes aromatisches Ringsystem" ein kondensiertes aromatisches Ringsystem mit im Allgemeinen 10 bis 20 Kohlenstoffatomen, bevorzugt 10 bis 14 Kohlenstoffatomen. Diese kondensierten aromatischen Ringsysteme können unsubstituiert oder mit linearen, verzweigten oder cyclischen C₁- bis C₂₀-, bevorzugt C₁- bis C₁₀-Alkylgruppen substituiert sein, die wiederum mit Halogen-, Nitro-, Ether- oder Carboxylgruppen substituiert sein können.

Des Weiteren können ein oder mehrere Kohlenstoffatome der Alkylgruppe durch Si, P, O, S oder N, bevorzugt O oder S, ersetzt sein. Des Weiteren können die kondensierten aromatischen Gruppen mit Halogen-, Nitro-, Carboxylgruppen, Aminogruppen oder Alkoxygruppen oder C₆- bis C₁₄-, bevorzugt C₆- bis C₁₀-Arylgruppen, insbesondere Phenyl- oder Naphthylgruppen, substituiert sein. Besonders bevorzugt bedeutet "kondensiertes aromatisches Ringsystem" ein kondensiertes aromatisches Ringsystem, das gegebenenfalls mit Halogen, bevorzugt Br, Cl oder F, Aminogruppen, bevorzugt NAr'Ar", wobei Ar und Ar' unabhängig voneinander C₆-Arlygruppen bedeuten, die, wie vorstehend definiert, unsubstituiert oder substituiert sein können oder Nitrogruppen substituiert ist. Ganz besonders bevorzugt ist das kondensierte aromatische Ringsystem unsubstituiert. Geeignete kondensierte aromatische Ringsysteme sind zum Beispiel Naphthalin, Anthracen, Pyren, Phenanthren oder Perylen.

Im Sinne der vorliegenden Anmeldung bedeutet heteroaromatischer Rest" eine C₄- bis C₁₄-, bevorzugt C₄- bis C₁₀-, besonders bevorzugt C₄- bis C₆-Heteroarylgruppe, enthaltend mindestens ein N- oder S-Atom. Diese Heteroarylgruppe kann unsubstituiert oder mit linearen, verzweigten oder cyclischen C₁- bis C₂₀-, bevorzugt C₁- bis C₉-Alkylgruppen, die wiederum mit Halogen-, Nitro-, Ether- oder Carboxylgruppen substituiert sein können, substituiert sein. Des Weiteren können ein oder mehrere Kohlenstoffatome der Alkylgruppe durch Si, P, O, S oder N, bevorzugt O oder S, ersetzt sein. Des Weiteren können die Heteroarylgruppen mit Halogen-, Nitro-, Carboxylgruppen, Aminogruppen oder Alkoxygruppen oder C₆- bis C₁₄-, bevorzugt C₆- bis C₁₀-Arylgruppen, substituiert sein. Unter Halogengruppen sind bevorzugt F, Cl oder Br zu verstehen. Besonders bevorzugt bedeutet "heteraromatischer Rest" eine Heteroarylgruppe, die gegebenenfalls mit Halogen, bevorzugt Br, Cl oder F, Aminogruppen, bevorzugt NArAr', wobei Ar und Ar' unabhängig voneinander C₆-Arlygruppen bedeuten, die, wie vorstehend definiert, unsubstituiert oder substituiert sein können, oder Nitrogruppen, substituiert ist. Ganz besonders bevorzugt ist die Heteroarylgruppe unsubstituiert.

Bevorzugt sind die Reste R⁴ und R⁵ in den Verbindungen der Formeln I, IIa, IIb und III H, Alkyl, Alkinyl, Aryloxy, besonders bevorzugt sind R⁴ und R⁵ H.

Die Reste R³ und R⁶ sind in den Verbindungen der Formeln I, IIa, IIb und III bevorzugt H, Alkyl, Alkinyl, Aryloxy, besonders bevorzugt sind R³ und R⁶ H.

Die Reste R¹ und R² in den Verbindungen der Formeln I, IIa, IIb und IV sind bevorzugt Alkylreste, besonders bevorzugt C₃-C₁₀-Alkylreste, ganz besonderes bevorzugt C₅-C₉-Alkylreste. Die Alkylreste können linear oder verzweigt sein, insbesondere sind sie linear.
- X¹ und X²: bedeuten bevorzugt jeweils Halogen, ausgewählt aus F, Cl, Br oder I, oder NO₂;
besonders bevorzugt sind X¹ und X² Cl oder Br.
- X³ und X⁴: bedeuten bevorzugt Halogen, ausgewählt aus F, Cl, Br und I, besonders bevorzugt Cl oder Br, verestertes Sulfonat oder einen Bor-haltigen Rest der Formel -B(O-[C(R⁷)₂]ₙ-O), wobei R⁷ gleich oder verschieden unabhängig voneinander H oder C₁-C₂₀-Alkyl und n eine ganze Zahl von 2 ist 10 bedeu- ten, besonders bevorzugt sind X³ uns X⁴ para-Toluolsulfonat (Tosylat), Triflat (F₃-SO₃), para-Nitrophenylsulfonat (nosylat), para-Bromsulfonat (Brosylat), ganz besonders bevorzugt Triflat, oder Bor-haltige Reste der Formel -B(O-[C(R⁷)₂]ₙ-O), wobei
R⁷ gleich oder verschieden ist und Wasserstoff oder C₁-C₂₀-Alkyl bedeutet, zum Beispiel Methyl, Ethyl, n-Propyl, iso-Propyl, n-Butyl, iso-Butyl, sec.- Butyl, tert-Butyl, n-Pentyl, iso-Pentyl, sec.-Pentyl, neo-Pentyl, 1,2- Dimethylpropyl, iso-Amyl, n-Hexyl, iso-Hexyl, sec.-Hexyl, n-Heptyl, iso- Heptyl, n-Octyl, n-Decyl, n-Dodecyl, oder n-Octadecyl; bevorzugt C₁-C₁₂- Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl oder n-Decyl, besonders bevor- zugt C₁-C₄-Alkyl wie Methyl, Ethyl, n-Propyl, iso-Propyl, n-Butyl, iso-Butyl, sec.-Butyl oder tert.-Butyl, ganz besonders bevorzugt Methyl; und n eine ganze Zahl von 2 bis 10, bevorzugt 2 bis 5 bedeutet. Ganz besonders bevorzugt sind Bor-haltige Reste der Formel -B(O-[C(CH₃)₂]₂)-O);
- X⁵ und X⁶: bedeuten bevorzugt Halogen, ausgewählt aus F, Cl, Br oder I, besonders bevorzugt Cl oder Br;
oder
verestertes Sulfonat oder einen Bor-haltigen Rest der Formel -B(O- [C(R⁷)₂]ₙ-O), wobei R⁷ gleich oder verschieden unabhängig voneinander H oder C₁-C₂₀-Alkyl und n eine ganze Zahl von 2 ist 10 bedeuten, besonders bevorzugt sind X⁵ und X⁶ para-Toluolsulfonat (Tosylat), Triflat (F₃-SO₃), para-Nitrophenylsulfonat (nosylat), para-Bromsulfonat (Brosylat), ganz be- sonders bevorzugt Triflat, oder Bor-haltige Reste der Formel -B(O-[C(R⁷)₂]ₙ- O), wobei
R⁷ gleich oder verschieden ist und Wasserstoff oder C₁-C₂₀-Alkyl bedeutet, zum Beispiel Methyl, Ethyl, n-Propyl, iso-Propyl, n-Butyl, iso-Butyl, sec.- Butyl, tert-Butyl, n-Pentyl, iso-Pentyl, sec.-Pentyl, neo-Pentyl, 1,2- Dimethylpropyl, iso-Amyl, n-Hexyl, iso-Hexyl, sec.-Hexyl, n-Heptyl, iso- Heptyl, n-Octyl, n-Decyl, n-Dodecyl, oder n-Octadecyl; bevorzugt C₁-C₁₂- Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl oder n-Decyl, besonders bevor- zugt C₁-C₄-Alkyl wie Methyl, Ethyl, n-Propyl, iso-Propyl, n-Butyl, iso-Butyl, sec.-Butyl oder tert.-Butyl, ganz besonders bevorzugt Methyl; und n eine ganze Zahl von 2 bis 10, bevorzugt 2 bis 5 bedeutet. Ganz besonders bevorzugt sind Bor-haltige Reste der Formel -B(O-[C(CH₃)₂]₂)-O).

Die Reste X¹ und X² bzw. X³ und X⁴ und X⁵ und X⁶ werden mit den folgenden Maßgaben ausgewählt:
- wenn X¹ und X² jeweils Halogen bzw. X³ und X⁴ Halogen, verestertes Sulfonat oder einen Bor-haltigen Rest bedeuten, bedeuten X⁵ und X⁶ ebenfalls Halogen, verestertes Sulfonat oder einen Bor-haltigen Rest, wobei die Reste X¹ und X² bzw. X³ und X⁴, und X⁵ und X⁶ so ausgewählt werden, dass das molare Verhältnis von Halogen und verestertem Sulfonat zu Bor-haltigem Rest 0,8 zu 1 bis 2,1 zu 1, bevorzugt 0,9 zu 1,1 bis 1,1 zu 0,9 beträgt, bevorzugt 1 : 1; oder, dass die Reste X¹ und X² in dem monomeren Fluoranthenderivat der Formel IIa jeweils Halogen bedeuten und gegebenenfalls mit weiteren Comonomeren umgesetzt werden, deren Reste X⁵ und X⁶ ebenfalls Halogen bedeuten. Im Fall eines nendcapping", bei dem nur die Endgruppen eines Polymers eingeführt werden müssen, reichen weit geringere Mengen an polymerisierbaren Gruppen aus (10-20 mol% bezogen auf die gesamte Monomerstoffmenge). Diese Endcapper sind allerdings monofunktionalisiert, um das Kettenwachstum zu beenden, d.h., es handelt sich um Verbindungen der Formel IIa oder IIb, die jeweils nur eine Gruppe X¹ oder X² (im Falle der Verbindungen der Formel IIa) bzw. X³ oder X⁴ (im Falle der Verbindungen der Formel IIb) tragen.

### Schritt a)

Die Herstellung der Fluoranthenderivate der allgemeinen Formel IIa erfolgt durch Umsetzung von Cyclopentanacenaphthenon-Derivaten (im Folgenden Acecyclonderivate genannt) der Formel III mit Alkinylverbindungen der Formel IV. Die Herstellung erfolgt in Analogie zu den in Dilthey et al., Chem. Ber. 1938, 71, 974 und Van Allen et al., J. Am. Chem. Soc., 1940, 62, 656 offenbarten Verfahren. Das Halogen-substituierte Acecyclonderivat der Formel III wird gemäß dem Stand der Technik bekannten Verfahren hergestellt. Halogenierte Acecyclonderivate sind zum Beispiel in JP 2000-007587 offenbart. Acecyclonderivate der Formel III, die Reste X¹ und X² tragen, deren Herstellung nicht im Stand der Technik offenbart ist, können in zu Verfahren gemäß dem Stand der Technik analogen Verfahren erhalten werden.

Die Alkinylverbindungen der Formel IV können ebenfalls nach dem Fachmann bekannten Verfahren hergestellt werden. Geeignete Verfahren sind zum Beispiel in Hagihara et al., Synthesis (1980), 627 und L. Cassar, J. Organometh. Chem. 93 (1979), 253 offenbart. Das molare Verhältnis des Acecyclonderivats der Formel III zu der Alkinylverbindung der Formel IV beträgt im Allgemeinen 1 : 1 bis 1,3 : 1, bevorzugt 1 : 1 bis 1,1:1.
Bei der Umsetzung gemäß Schritt a) des erfindungsgemäßen Verfahrens handelt es sich um eine Diels-Alder-Reaktion mit anschließender Kohlenmonoxid-Eliminierung.

Die Umsetzung erfolgt im Allgemeinen in einem Lösungsmittel, bevorzugt in einem organischen unpolaren Lösungsmittel, besonders bevorzugt in einem organischen unpolaren Lösungsmittel mit einem Siedepunkt oberhalb von im Allgemeinen 100 °C, bevorzugt oberhalb von 140 °C, besonders bevorzugt oberhalb von 260 °C.

Geeignete Lösungsmittel sind zum Beispiel Toluol, Xylol, Diphenylether, Methylnaphthalin, Mesitylen, Glykole und ihre Ether, Dekalin und Mischungen der genannten Lösungsmittel.

In einer bevorzugten Ausführungsform des erfindungsgemäßen Verfahrens werden das Acecyclonderivat der Formel III und die Alkinylverbindung der Formel IV in dem organischen Lösungsmittel zusammen gegeben und auf Temperaturen von im Allgemeinen 140 bis 260 °C, bevorzugt 140 bis 170 °C bzw. 240 bis 260 °C erhitzt. Die Temperatur ist dabei abhängig von der Reaktivität der Ausgangsstoffe. Terminale Alkine (R² = H in Formel IV) reagieren im Allgemeinen bei niedrigeren Temperaturen, bevorzugt bei 140 bis 190 °C, besonders bevorzugt bei 140 bis 170 °C, ganz besonders bevorzugt 140 bis 160 °C, während interne Alkine (R² ≠ H in Formel IV) im Allgemeinen bei höheren Temperaturen reagieren, bevorzugt bei 190 bis 260 °C, besonders bevorzugt 220 bis 260 °C, ganz besonders bevorzugt 240 bis 260 °C.

Die Reaktionsdauer beträgt im Allgemeinen 8 bis 30 Stunden. Die Reaktionsdauer hängt von dem sterischen Anspruch von R¹ und R² in Formel IV ab. Bevorzugt beträgt die Reaktionsdauer 8 bis 18, besonders bevorzugt 10 bis 16, ganz besonders bevorzugt 14 bis 16 Stunden.

Das erhaltene Reaktionsgemisch wird in einem polaren Lösungsmittel, zum Beispiel in Methanol, Ethanol, oder gegebenenfalls in einem unpolaren Lösungsmittel wie Cyclohexan gefällt. Bei besonders löslichen Fluoranthenderivaten kann der Fällungsschritt entfallen. Das erhaltene Produkt wird nach dem Fachmann bekannten Methoden aufgearbeitet. Bevorzugt erfolgt die Aufarbeitung durch Säulenchromatographie. Als Laufmittel kann jedes geeignete Laufmittel oder Laufmittelgemisch eingesetzt werden. Ganz besonders bevorzugt wird ein Essigester/Cyclohexangemisch eingesetzt.

Die erhaltenen halogenierten Fluoranthenderivate der Formel IIa können entweder direkt in der Polymerisation in Schritt c) eingesetzt werden oder zu Fluoranthenderivaten der Formel IIb in Schritt b) umgesetzt werden, in Abhängigkeit davon, welche polymerisierbaren Gruppen X¹, X² bzw. X³, X⁴ gewünscht sind.

### Schritt b)

Schritt b) ist optional und wird nur dann durchgeführt, wenn Fluoranthenderivate der Formel IIb in der darauf folgenden Polymerisation (Schritt c)) eingesetzt werden.

Die Überführung der Halogenidreste X¹ und X² in dem monomeren Fluoranthenderivat der Formel IIa in die Reste X³ und X⁴ ausgewählt aus Halogen, verestertem Sulfonat und Bor-haltigem Rest, wie vorstehend erwähnt, gemäß Formel IIb, erfolgt nach dem Fachmann bekannten Verfahren. Es ist darauf hinzuweisen, dass X³ oder X⁴ in den Fällen Halogen sein können, wenn der jeweils andere Rest nicht Halogen ist. Fluoranthenderivate, worin beide polymerisierbaren Gruppen gleichzeitig Halogen sind, sind bereits von den Fluoranthenderivaten der Formel IIa umfasst.

Die Herstellung von Fluoranthenderivaten der Formel IIb, worin X³ und/oder X⁴ einen Bor-haltigen Rest der Formel -B(O-[C(R⁷)₂]ₙ-O) bedeuten, erfolgt zum Beispiel durch Metallierung von Verbindungen der Formel IIa, worin X¹ und X² Halogen, bevorzugt Chlor oder Brom, besonders bevorzugt Brom, bedeuten. Zunächst erfolgt eine Umsetzung mit mindestens zwei oder mindestens vier Äquivalenten einer starken metallorganischen Base, wodurch einfach bzw. zweifach metalliert wird, und anschließend mit einem bzw. zwei Äquivalenten einer Borverbindung der allgemeinen Formel X⁷-B(O-[C(R⁷)₂]ₙ-O) umgesetzt wird, wobei R⁷ gleich oder verschieden und unabhängig voneinander H oder C₁-C₂₀-Alkyl, n eine ganze Zahl von 2 bis 10 und X⁷ C₁-C₆-Alkoxy wie Methoxy, Ethoxy, n-Propoxy, iso-Propoxy, n-Butoxy, iso-Butoxy, sec.-Butoxy, tert.-Butoxy, n-Penthoxy, iso-Penthoxy, n-Hexoxy und iso-Hexoxy, bevorzugt Methoxy, Ethoxy, n-Propoxy, iso-Propoxy und n-Butoxy, besonders bevorzugt iso-Propoxy, bedeuten. Bevorzugte Reste R⁷ und Definitionen für n wurden bereits vorstehend genannt.

Als Base können die in der metallorganischen Chemie gängigen Metallalkyle verwendet werden, zum Beispiel Methyllithium, Ethyllithium, n-Butyllithium, sec-Butyllithium, tert-Butyllithium oder Hexyllithium; oder Grignard-Verbindungen, zum Beispiel Ethylmagnesiumbromid. Als Lösungsmittel sind hochsiedende Lösungsmittel wie Toluol, ortho-Xylol, metha-Xylol, para-Xylol, Ethylbenzol oder Mischungen davon geeignet. Des Weiteren sind als Lösungsmittel nicht-cyclische oder cyclische Ether wie 1,2-Diemethoxyethan, Tetrahydrofuran, Dioxan oder Diethylether geeignet.

Die Reaktionsdauer der Metallierung beträgt im Allgemeinen von 1 bis 10 Stunden, bevorzugt 2 bis 5 Stunden. Die Temperaturbedingungen sind im Allgemeinen unkritisch, bevorzugt erfolgt die Metallierung bei Temperaturen von -90 °C bis -20 °C.

Anschließend setzt man die einfach oder zweifach metallierte Verbindung mit mindestens einem bzw. zwei Äquivalenten der vorstehend genannten Borverbindung um. Dazu mischt man die beiden Komponenten miteinander in einem geeigneten Lösungsmittel wie Benzol, Toluol, Ethylbenzol, ortho-Xylol, meta-Xylol oder para-Xylol, Chlorbenzol, Cyclohexan, Acetonitril, Tetrahydrofuran, 1,2-Dimethoxyethan, Dioxan oder Diethylether oder Mischungen davon. Diese Umsetzung wird im Allgemeinen bei Temperaturen von -100 °C bis 150 °C, bevorzugt -78 °C bis +100 °C durchgeführt. Wichtig ist, dass die Umsetzung unter Ausschluss von Sauerstoff und Feuchtigkeit durchgeführt wird. Die Druckbedingungen sind im Allgemeinen unkritisch, bevorzugt erfolgt die Umsetzung bei Atmosphärendruck. Die Reaktionsdauer beträgt im Allgemeinen 10 Minuten bis 2 Tage, bevorzugt 1 Stunde bis 24 Stunden.

Die Aufarbeitung und Reinigung der monomeren Fluoranthenderivate IIb, worin X³ und/oder X⁴ einen Bor-haltigen Rest bedeuten, kann nach konventionellen Methoden erfolgen, beispielsweise durch Extraktion, Perforation, Kristallisation, Chromatographie, Umfällen oder Sublimation.

Die vorstehend genannten Bor-Verbindungen der Formel X⁷-B(O-[C(R⁷)ₙ]-O können nach dem Fachmann bekannten Verfahren hergestellt werden, einige Derivate sind kommerziell erhältlich.

Eine weitere Möglichkeit, halogenierte Aromaten in die entsprechenden Boronester zu überführen, ist die Umsetzung mit den Diboronen (OR)₂B-B(OR)₂ unter Einsatz eines Palladiumkatalysators wie PdCl₂(dppf) nach Miyaura, z.B. beschrieben in J. Org. Chem. 1995, 60, 7508.

Die Herstellung von Fluoranthenderivaten der Formel IIb, worin X³ und/oder X⁴ verestertes Sulfonat bedeuten, kann ausgehend von Fluoranthenderivaten der Formel IIa erfolgen, worin X¹ und/oder X² eine Nitrogruppe sind. Zur Umwandlung der Nitrogruppe in eine veresterte Sulfonatgruppe kann zum Beispiel eine Reduktion der Nitrogruppe zur Aminogruppe, Diazotierung der Aminogruppe mit NaNO₂/HCl oder Amylnitrit und Verkochen der Diazoniumsalze und abschließende Umsetzung der erhaltenen phenolischen OH-Gruppen mit den entsprechenden Chlorsulfonsäuregruppen, zum Beispiel CF₃SO₂Cl (wobei sich ein Triflat ergibt) oder para-CH₃-C₆H₄-SO₂Cl (wobei ein Tosylat erhalten wird), durchgeführt werden.

Eine weitere Möglichkeit ist die Umsetzung eines aromatischen Diazoniumsalzes, das wie vorstehend synthetisiert wurde, mit einem halogenierten Aromaten wie beispielsweise von Wegner et al. beschrieben in Chem. Eur. J. 2004, 10, 2681. In Chem. Eur. J. 2004, 10, 2681 ist die Synthese eines aromatischen Diazoniumsalzes und die Bedingungen einer C-C-Knüpfung eines solchen Diazoniumalzes mit einem aromatischen Boronester beschrieben.

### Schritt c)

Die monomeren Fluoranthenderivate der Formeln IIa und/oder IIb werden, gegebenenfalls gemeinsam mit mindestens einem weiteren Comonomeren, ausgewählt aus der Gruppe bestehend aus einem weiteren von dem ersten Fluoranthenderivat der Formel IIa oder IIb verschiedenen Fluoranthenderivat der Formel IIa oder IIb, aromatischen, kondensierten aromatischen und heteroaromatischen Verbindungen, die jeweils zwei mit den Gruppen X¹ und X² der Fluoranthenderivate der Formel IIa oder den Gruppen X³ oder X⁴ der Fluoranthenderivate der Formel IIb polymerisierbare Gruppen X⁵ und X⁶ aufweisen.

Grundsätzlich kann die Polymerisation in Schritt c) in Abhängigkeit von den polymerisierbaren Gruppen der monomeren Fluoranthenderivate X¹ und X² bzw. X³ und X⁴ bzw. den polymerisierbaren Gruppen der gegebenenfalls eingesetzten weiteren Comonomere X⁵ und X⁶ mit Hilfe jedes geeigneten Polymerisationsverfahrens erfolgen. Geeignete Polymerisationsverfahren und die dazu erforderlichen polymerisierbaren Gruppen sind zum Beispiel in EP-A 1 245 659 (Seiten 26 bis 31) aufgeführt.

In einer bevorzugten Ausführungsform erfolgt die Polymerisation der Fluoranthenderivate der Formel IIa und/oder IIb, gegebenenfalls mit mindestens einem weiteren Comonomeren, z. B. durch Yamamoto-Kupplung oder durch Suzuki-Reaktion, in Gegenwart von Nickel- oder Palladiumverbindungen.

In dieser Ausführungsform bedeuten
- X¹ und X²: jeweils Halogen, ausgewählt aus F, Cl, Br und I; bzw.
- X³ und X⁴: Halogen, ausgewählt aus F, Cl, Br und I, verestertes Sulfonat oder einen Bor-haltigen Rest der Formel -B(O-[C(R⁷)₂]ₙ-O und
- X⁵ und X⁶: Halogen, ausgewählt aus F, Cl, Br und I, verestertes Sulfonat oder einen Bor-haltigen Rest der Formel -B(O-[C(R⁷)₂]ₙ-O;
- R⁷: gleich oder verschieden unabhängig voneinader H oder C₁-C₂₀-Alkyl;
- n: eine ganze Zahl von 2 bis 10;
wobei die Reste X¹ und X² bzw. X³ und X⁴ sowie X⁵ und X⁶ so ausgewählt werden, dass das molare Verhältnis von Halogen und verestertem Sulfonat zu Bor-haltigem Rest 0,8 zu 1 bis 2,1 zu 1, bevorzugt 0,9 zu 1,1 bis 1,1 zu 0,9 beträgt, bevorzugt 1 : 1; oder, dass die Reste X¹ und X² in dem monomeren Fluoranthenderivat der Formel IIa jeweils Halogen bedeuten und gegebenenfalls mit weiteren Comonomeren umgesetzt werden, deren Reste X⁵ und X⁶ ebenfalls Halogen bedeuten. Im Fall eines "endcapping", bei dem nur die Endgruppen eines Polymers eingeführt werden müssen, reichen weit geringere Mengen an polymerisierbaren Gruppen aus (10-20 mol% bezogen auf die gesamte Monomerstoffmenge). Diese Endcapper sind allerdings monofunktionalisiert, um das Kettenwachstum zu beenden, d.h., es handelt sich um Verbindungen der Formel IIa oder IIb, die jeweils nur eine Gruppe X¹ oder X² (im Falle der Verbindungen der Formel IIa) bzw. X³ oder X⁴ (im Falle der Verbindungen der Formel IIb) tragen;
oder, dass die Reste X¹ und X² in dem monomeren Fluoranthenderivat jeweils Halogen bedeuten und diese gegebenenfalls gemeinsam mit weiteren Comonomeren umgesetzt werden, worin die Reste X⁵ und X⁶ ebenfalls jeweils Halogen bedeuten. D. h., bevorzugt erfolgt entweder eine Umsetzung von monomeren Fluoranthenderivaten gegebenenfalls mit weiteren Comonomeren, worin alle polymerisierbaren Gruppen X¹, X² und gegebenenfalls X⁵ und X⁶ Halogen, bedeuten. In diesem Fall wird als Katalysator bevorzugt eine Nickelverbindung eingesetzt. Oder es erfolgt eine Umsetzung von monomeren Fluoranthenderivaten und gegebenenfalls weiteren Comonomeren, wobei die polymerisierbaren Gruppen X¹, X² bzw. X³, X⁴ und gegebenenfalls X⁵ und X⁶ Halogen oder veresters Sulfonat auf der einen Seite und Bor-haltige Reste auf der anderen Seite in den angegebenen molaren Verhältnissen bedeuten. Bei dieser Umsetzung wird jeweils Halogen oder verestertes Sulfonat mit einem Bor-haltigen Rest umgesetzt. In diesem Fall wird bevorzugt eine Palladiumverbindung als Katalysator eingesetzt.

Bevorzugte Definitionen für X¹, X², X³, X⁴, X⁵, X⁶, R⁷ und n wurden bereits genannt.

Bevorzugt erfolgt die Polymerisation in Schritt c) in dieser Ausführungsform des erfindungsgemäßen Verfahrens in Gegenwart einer Nickel- oder Palladiumverbindung, die insbesondere in der Oxidationsstufe 0 vorliegt. Bevorzugt werden das kommerziell erhältliche Tetrakis(triphenylphosphan)-palladium [Pd(P(P₆H₅)₃)₄] sowie kommerziell erhältliche Nickelverbindungen, zum Beispiel Ni(C₂H₄)₃, Ni(1,5-cyclooctadien)₂ ("Ni(COD)₂"), Ni(1,6-cyclodecadien)₂ oder Ni(1,5,9-all-trans-cyclododecadien)₂ eingesetzt. Ganz besonders bevorzugt werden [Pd(P(C₆H₅)₃)₄] und Ni(COD)₂ eingesetzt. Zur Durchführung der Polymerisation kann man einen Überschuss an P(C₆H₅)₃ bzw. 1,5-Cyclooctadien, in Abhängigkeit des eingesetzten Katalysators, zugeben.

Bei der Durchführung der Polymerisation in Gegenwart von Palladiumverbindungen reichen üblicherweise katalytische Mengen, das heißt 0,1 bis 10 mol-% Pd, bezogen auf das monomere Fluoranthenderivat der Formel IIa bzw. IIb. Erfolgt die Polymerisation in Gegenwart von Nickel-Verbindungen, so werden üblicherweise stöichiometrische Mengen an Ni, bezogen auf die Summe der monomeren Fluoranthenderivate der Formel IIa oder IIb, gegebenenfalls gemeinsam mit weiteren Comonomeren, eingesetzt.

Die Polymerisation wird üblicherweise in einem organischen Lösungsmittel durchgeführt, zum Beispiel in Toluol, Ethylbenzol, meta-Xylol, ortho-Xylol, Dimethylformamid (DMF), Tetrahydrofuran, Dioxan oder Mischungen der vorstehend genannten Lösungsmittel. Das oder die Lösungsmittel werden vor der Polymerisation nach üblichen Methoden von Spuren von Feuchtigkeit befreit.

Die Polymerisation wird üblicherweise unter Schutzgas durchgeführt. Geeignet sind Stickstoff, CO₂ oder Edelgase, insbesondere Argon oder CO₂.

Üblicherweise führt man die Polymerisation in Gegenwart einer Base durch, zum Beispiel sind organische Amine geeignet, besonders geeignet sind Triethylamin, Pyridin oder Collidin.

Die Polymerisation kann auch in Gegenwart von festen basischen Salzen, zum Beispiel Alkalimetallcarbonat oder Alkalimetallbicarbonat, gegebenenfalls in Gegenwart eines Kronenethers wie 18-Kronen-6 durchgeführt werden. Es ist ebenfalls möglich, die Polymerisation als Zweiphasenreaktion mit wässrigen Lösungen von Alkalimetallcarbonat, gegebenenfalls in Gegenwart eines Phasentransferkatalysators, durchzuführen. In diesem Falle ist es nicht nötig, das organische Lösungsmittel vor der Reaktion von Feuchtigkeit zu befreien.

Üblicherweise dauert die Polymerisation zwischen 10 Minuten und bis zu 2 Tagen, bevorzugt 2 Stunden bis 24 Stunden. Die Druckbedingungen sind unkritisch, bevorzugt ist Atmosphärendruck. Im Allgemeinen führt man die Polymerisation bei erhöhter Temperatur durch, bevorzugt zwischen 80 °C und dem Siedepunkt des organischen Lösungsmittels bzw. Lösungsmittelgemisches, besonders bevorzugt zwischen 100 °C und dem Siedepunkt des organischen Lösungsmittels bzw. Lösungsmittelgemisches. Das Molverhältnis der Summe von Halogen und verestertem Sulfonat einerseits und Bor-haltigen Resten andererseits in den eingesetzten monomeren Fluoranthenderivaten der Formel IIa und/oder IIb bzw. den eingesetzten weiteren Comonomeren beträgt 0,8 zu 1 bis 1,2 zu 1, bevorzugt 0,9 zu 1,1 bis 1,1 zu 0,9 beträgt, bevorzugt 1 : 1. Im Fall eines "endcapping", bei dem nur die Endgruppen eines Polymers eingeführt werden müssen, reichen weit geringere Mengen an polymerisierbaren Gruppen aus (10-20 mol% bezogen auf die gesamte Monomerstoffmenge). Diese Endcapper sind allerdings monofunktionalisiert, um das Kettenwachstum zu beenden, d.h., es handelt sich um Verbindungen der Formel IIa oder IIb, die jeweils nur eine Gruppe X¹ oder X₂ (im Falle der Verbindungen der Formel IIa) bzw. X³ oder X⁴ (im Falle der Verbindungen der Formel IIb) tragen.

Geeignete weitere Comonomere ausgewählt aus der Gruppe bestehend aus aromatischen, kondensierten aromatischen und heteroaromatischen Verbindungen, die jeweils zwei mit den Gruppen X¹ und X² des Fluoranthenderivats der Formel IIa bzw. den Gruppen X³ und X⁴ des Fluorantehnderivats der Formel IIb polymerisierbare Gruppen X⁵ und X⁶ aufweisen, sind insbesondere die entsprechenden Phenylen-Verbindungen, (E-) oder (Z)-Ethylenverbindungen, Acetylenverbindungen, Naphthylenverbindungen, Anthrylenverbindungen Arylaminoverbindungen, Fluorenderivate, Carbazolderivate, Dibenzofuranderivate, Pyrenderivate, Phenanthrenderivate und/oder Thiophenverbindungen, die die bereits erwähnten Reste X⁵ und X⁶ aufweisen.

Insbesondere sind als weitere Monomere aromatische, kondensierte aromatische und heteroaromatische Verbindungen geeignet, die neben den polymerisationsfähigen Gruppen X⁵ und X⁶ solubilisierende Alkyl- oder Alkoxyseitenketten tragen, zum Beispiel eine oder zwei C₃-C₁₀-Alkyl- und/oder C₃-C₁₀-Alkoxyseitenketten.

Besonderes bevorzugte weitere Comonomere ausgewählt aus der Gruppe bestehend aus aromatischen, kondensierten aromatischen und heteroaromatischen Verbindungen, die jeweils zwei mit den Gruppen X¹ und X² des Fluoranthenderivats der Formel IIa bzw. den Gruppen X³ und X⁴ des Fluoranthenderivats der Formel IIb polymerisierbare Gruppen X⁵ und X⁶ aufweisen, und die zum Einsatz in der vorstehend aufgeführten bevorzugten Ausführungsform des Polymerisationsschritts c) des erfindungsgemäßen Verfahrens geeignet sind, sind:
Phenylenbisboronsäuren bzw. ihre Ester, bevorzugt 1,4-Phenylenbisboronsäuren bzw. ihre Ester, und ihre alkyl- oder alkoxysubstituierten Derivate,
dihalogensubstituierte Benzole, bevorzugt 1,4-dihalogensubstituierte Benzole, und ihre alkyl- oder alkoxysubstituierten Derivate,
Anthracenbisboronsäuren bzw. ihre Ester, bevorzugt 1,5- oder 9,10-Anthracenbisboronsäuren bzw. ihre Ester, und Dihaloanthracen, bevorzugt 1,5- oder 9,10-Dihaloanthracen,
dihalogensubstituierte Triarylamine und ihre Bisboronsäuren bzw. ihre Ester und ihre alkyl- oder alkoxysubstituierten Derivate,
dihalogensubstituierte Naphthaline und ihre Bisboronsäuren bzw. ihre Ester und ihre alkyl- oder alkoxysubstituierten Derivate, im besonderen 1,5-Dialkoxy-2,6-dibromonaphthalin,
dihalogensubstituierte Fluorene und ihre Bisbornosäuren bzw. ihre Ester und ihre alkyl- oder alkoxysubstituierten Derivate,
dihalogensubstituierte Carbazole und ihre Bisboronsäuren bzw. ihre Ester und ihre alkyl- oder alkoxysubstituierten Derivate,
dihalogensubstitueirte Dibenzofurane und ihre Bisboronsäuren bzw. ihre Ester und ihre alkyl- oder alkoxysubstituerten Derivate,
dihalogensubstituierte Pyrene und ihre Bisboronsäuren bzw. ihre Ester und ihre alkyl- oder alkoxysubstituierten Derivate,
dihalogensubstituierte Phenanthrene und ihre Bisboronsäure bzw. ihre Ester und ihre alkyl- oder alkoxysubstituierten Derivate.

Besonders bevorzugt ist das mindestens eine weitere Comonomer ausgewählt aus der Gruppe bestehend aus Phenylenbisboronsäuren, Phenylenbisbornosäureestern und dihalogensubstituierten Triarylaminen.

Geeignete Alkyl- oder Alkoxysubstituenten sind C₃-C₁₀-Alkyl- oder C₃-C₁₀-Alkoxyseitenketten, wobei die vorstehend genannten Verbindungen bevorzugt gegebenenfalls einen oder zwei Alkyl- oder Alkoxysubstituenten tragen.

Die erfindungsgemäßen Polyfluoranthene können gegebenenfalls weiter vernetzt werden, insbesondere, wenn Sie mindestens einen Rest R¹, R², R³, R⁴, R⁵ oder R⁶ aufweisen, der eine der nachfolgenden Bedeutungen aufweist:
- CH=CH₂, -C≡CH, (E)- oder (Z)- CH=CH-C₆H₅, Acryloyl, Methacryloyl, ortho-Methylstyryl, para-Methylstyryl, -O-CH=CH₂, Glycidyl, 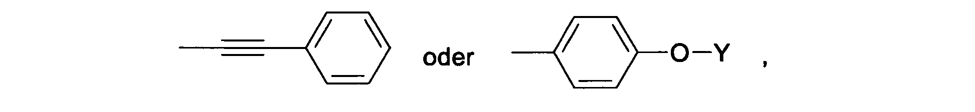 wobei Y Acryloyl, Methacryloyl, ortho- oder para-Methylstyryl, -O-CH=CH₂ oder Glycidyl bedeutet.

Die Vernetzung kann photochemisch in Anwesenheit eines Initiators oder thermisch durchgeführt werden.

Eine thermische Vernetzung wird bevorzugt so durchgeführt, dass die erfindungsgemäßen Polyfluoranthene, wobei mindestens ein Rest R¹ bis R⁶ die vorstehend genannten Bedeutungen aufweist, in Substanz oder in einem Lösungsmittel unter Einwirkung von Wärme, bevorzugt 40 bis 100 °C unter Inertgas, im Allgemeinen Stickstoff oder Edelgas, erhitzt werden. Bevorzugt erfolgt die Vernetzung im Zuge der OLED-Präparation, nach Aufringen der Polyfluoranthenschicht. Besonders bevorzugt werden die erfindungsgemäßen Polyfluoranthene in Substanz oder in einem Lösungsmittel als Film aufgetragen, bevorzugt auf eine der Elektroden (auf eine Lochtransportschicht wie z.B. PEDOT auf ITO-Anode) des OLEDs, und über einen Zeitraum von im Allgemeinen 45 Minuten bis 90 Minuten unter Stickstoff oder Edelgas erhitzt. Der bevorzugte Temperaturbereich ist bereits vorstehend angegeben. Die Durchführung von thermischen Polymerisationen ist dem Fachmann bekannt.

Besonders bevorzugt sind die Reste R¹, R², R³, R⁴, R⁵ oder R⁶ bei Durchführung einer thermischen Vernetzung unabhängig voneinander (E)- oder (Z)- CH=CH-C₆H₅, ortho-Methylstyryl, para-Methylstyryl oder 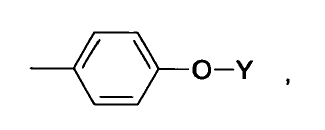 und Y ist bevorzugt (E)- oder (Z)- CH=CH-C₆H₅, ortho-Methylstyryl oder para-Methylstyryl.

Die photochemische Vernetzung wird bevorzugt durchgeführt, in dem das erfindungsgemäße Polyfluoranthenderivat, worin mindestens einer der Reste R¹ bis R⁶ die vorstehend angegebenen Bedeutungen aufweist, in Substanz oder in Lösung in Gegenwart eines gängigen Photoinitiators, der aus der Photopolymerisation von zum Beispiel Acrylsäurederivaten oder Methacrylsäurederivaten oder ungesättigten Ethern dem Fachmann bekannt ist, mit einer Strahlenquelle, zum Beispiel einer UV-Lampe, belichtet. Bevorzugt erfolgt die Vernetzung im Zuge der OLED-Präparation, nach Aufbringen der Polyfluoranthenschicht. Besonders bevorzugt werden die erfindungsgemäßen Polyfluoranthene in Substanz oder in einem Lösungsmittel als Film aufgetragen, bevorzugt auf eine der Elektroden (auf eine Lochtransportschicht wie z.B. PEDOT auf ITO-Anode) des OLEDs, und in Gegenwart eines gängigen Photoinitiators, mit einer Strahlenquelle, zum Beispiel einer UV-Lampe, belichtet. Die Reaktionsbedingungen von Photopolymerisationen sind dem Fachmann bekannt und z.B. in EP-A 0 637 889 offenbart.

Bevorzugt sind die Reste R¹, R², R³, R⁴, R⁵ oder R⁶ bei Durchführung einer photochemischen Polymerisation oder Photopolymerisation unabhängig voneinander Acryloyl, Methacryloyl, -O-CH=CH₂, Glycidyl oder 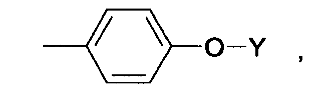 und Y bedeutet Acryloyl, Methacryloyl, -O-CH=CH₂ oder Glycidyl.

Ein weiterer Gegenstand der vorliegenden Erfindung sind Polyfluoranthene herstellbar nach dem erfindungsgemäßen Verfahren. Dabei werden - je nach Ausführungsform der Erfindung - unterschiedliche Polyfluoranthene zugänglich. Allen Polyfluoranthenen gemeinsam sind deren Elektrolumineszenzeigenschaften, so dass die Polyfluoranthene zum Einsatz in OLEDs geeignet sind. Bevorzugte Ausführungsformen des erfindungsgemäßen Verfahrens sowie der Reste der eingesetzten Verbindungen wurden bereits vorstehend genannt.

Dadurch, dass es möglich ist, Filme aus den erfindungsgemäßen Polyfluoranthenen zu bilden, ist eine Aufbringung der Polyfluoranthene auf Elektroden in einem OLED aus Lösung möglich, zum Beispiel durch Spincoating oder Tauchen, was es ermöglicht, großflächige Displays einfach und kostengünstig herzustellen.

Ein weiterer Gegenstand der vorliegenden Erfindung sind somit Filme enthaltend oder bestehend aus den erfindungsgemäßen Polyfluoranthenen bzw. Polyfluoranthenen, die gemäß dem erfindungsgemäßen Verfahren hergestellt werden.

Des Weiteren betrifft die vorliegende Erfindung organische Leuchtdioden (OLEDs) enthaltend mindestens ein erfindungsgemäßes Polyfluoranthen.

Organische Leuchtdioden (OLEDs) sind grundsätzlich aus mehreren Schichten aufgebaut:
1. Anode
2. Löcher-transportierende Schicht
3. Licht-emittierende Schicht
4. Elektronen-transportierende Schicht
5. Kathode

Die erfindungsgemäßen Polyfluoranthene werden bevorzugt in der Licht-emittierenden Schicht als Emittermoleküle eingesetzt. Ein weiterer Gegenstand der vorliegenden Anmeldung ist daher eine Licht-emittierende Schicht enthaltend oder bestehend aus mindestens einem erfindungsgemäßen Polyfluoranthen.

Die erfindungsgemäßen Polyfluoranthene liegen im Allgemeinen in Substanz - ohne weitere Zusätze - in der Licht-emittierenden Schicht vor. Es ist jedoch ebenfalls möglich, dass neben den erfindungsgemäßen Polyfluoranthenen weitere Verbindungen in der Licht-emittierenden Schicht vorliegen. Beispielsweise kann ein fluoreszierender Farbstoff anwesend sein, um die Emissionsfarbe des als Emittersubstanz eingesetzten Polyfluoranthens zu verändern. Des Weiteren kann ein Verdünnungsmaterial eingesetzt werden. Dieses Verdünnungsmaterial kann ein Polymer sein, zum Beispiel Poly(N-vinylcarbazol) oder Polysilan. Das Verdünnungsmaterial kann jedoch des Weiteren ein kleines Molekül sein, zum Beispiel 4,4'-N,N'-Dicarbazolbiphenyl (CDP) oder tertiäre aromatische Amine. Wenn ein Verdünnungsmaterial eingesetzt wird, beträgt der Anteil der erfindungsgemäßen Polyfluoranthene in der Licht-emittierenden Schicht im Allgemeinen weniger als 20 Gew.-%. Üblicherweise wird kein Verdünnungsmaterial eingesetzt.

Die einzelnen der vorstehend genannten Schichten des OLEDs können wiederum aus 2 oder mehreren Schichten aufgebaut sein. Beispielsweise kann die Löcher-transportierende Schicht aus einer Schicht aufgebaut sein in die aus der Elektrode Löcher injiziert werden und einer Schicht, die die Löcher von der Loch injizierenden Schicht weg in die Licht-emittierende Schicht transportiert. Die Elektronen-transportierende Schicht kann ebenfalls aus mehreren Schichten bestehen, zum Beispiel einer Schicht, worin Elektronen durch die Elektrode injiziert werden, und einer Schicht, die aus der Elektronen-injizierenden Schicht Elektronen erhält und in die Licht-emittierende Schicht transportiert. Diese genannten Schichten werden jeweils nach Faktoren wie Energieniveau, Temperaturresistenz und Ladungsträgerbeweglichkeit, sowie Energiedifferenz der genannten Schichten mit den organischen Schichten oder den Metallelektroden ausgewählt. Der Fachmann ist in der Lage, den Aufbau der OLEDs so zu wählen, dass er optimal an die erfindungsgemäß als Emittersubstanzen verwendeten Polyfluoranthene angepasst ist.

Um besonders effiziente OLEDs zu erhalten, sollte das HOMO (höchstes besetztes Molekülorbital) der Loch-transportierenden Schicht mit der Arbeitsfunktion der Anode angeglichen sein und das LUMO (niedrigstes unbesetztes Molekülorbital) der Elektronen-transportierenden Schicht sollte mit der Arbeitsfunktion der Kathode angeglichen sein.

Ein weiterer Gegenstand der vorliegenden Anmeldung ist ein OLED enthaltend mindestens eine erfindungsgemäße Licht-emittierende Schicht. Die weiteren Schichten in dem OLED können aus einem beliebigen Material aufgebaut sein, das üblicherweise in solchen Schichten eingesetzt wird und dem Fachmann bekannt ist.

Die Anode (1) ist eine Elektrode, die positive Ladungsträger bereitstellt. Sie kann zum Beispiel aus Materialien aufgebaut sein, die ein Metall, eine Mischung verschiedener Metalle, eine Metalllegierung, ein Metalloxid oder eine Mischung verschiedener Metalloxide enthält. Alternativ kann die Anode ein leitendes Polymer sein. Geeignete Metalle umfassen die Metalle der Gruppen Ib, IVa, Va und VIa des Periodensystems der Elemente sowie die Übergangsmetalle der Gruppe VIII. Wenn die Anode lichtdurchlässig sein soll, werden im Allgemeinen gemischte Metalloxide der Gruppen IIb, IIIb und IVb des Periodensystems der Elemente eingesetzt, zum Beispiel Indium-Zinn-Oxid (ITO). Es ist ebenfalls möglich, dass die Anode (1) ein organisches Material, zum Beispiel Polyanilin enthält, wie beispielsweise in Nature, Vol. 357, Seiten 477 bis 479 (11. Juni 1992) beschrieben ist. Zumindest entweder die Anode oder die Kathode sollten mindestens teilweise transparent sein, um das gebildete Licht auskoppeln zu können.

Geeignete Lochtransportmaterialien für die Schicht (2) des erfindungsgemäßen OLEDs sind zum Beispiel in Kirk-Othmer Encyclopedia of Chemical Technologie, 4. Auflage, Vol. 18, Seiten 837 bis 860, 1996 offenbart. Sowohl Löcher transportierende Moleküle als auch Polymere können als Lochtransportmaterial eingesetzt werden. Üblicherweise eingesetzte Löcher transportierende Moleküle sind ausgewählt aus der Gruppe bestehend aus 4,4'-Bis[N-(1-naphthyl)-N-phenyl-amino]biphenyl (α-NPD), N,N'-Diphenyl-N,N'-bis(3-methylphenyl)-[1,1'-biphenyl]-4,4'-diamin (TPD), 1,1-Bis[(di-4-tolylamino)-phenyl]cyclohexan (TAPC), N,N'-Bis(4-methylphenyl)-N,N'-Bis(4-ethylphenyl)-[1,1'-(3,3'-dimethyl)biphenyl]-4,4'-diamin (ETPD), Tetrakis-(3-methylpheny))-N,N,N',N'-2,5-phenylendiamin (PDA), α-Phenyl-4-N,N-diphenylaminostyrol (TPS), p-(Diethylamino)-benzaldehyddiphenylhydrazon (DEH), Triphenylamin (TPA), Bis[4-(N,N-diethylamino)-2-methylphenyl)(4-methyl-phenyl)methan (MPMP), 1-Phenyl-3-[p-(diethylamino)styryl]-5-[p-(diethylamino)phenyl]pyrazolin (PPR oder DEASP), 1,2-trans-Bis(9H-carbazol-9-yl)cyclobutan (DCZB), N,N,N',N'-Tetrakis(4-methylphenyl)-(1,1'-biphenyl)-4,4'-diamin (TTB) und Porphyrinverbindungen wie Kupferphthalocyanine. Üblicherweise eingesetzte Löcher transportierende Polymere sind ausgewählt aus der Gruppe bestehend aus Polyvinylcarbazolen, (Phenylmethyl)polysilanen und Polyanilinen. Es ist ebenfalls möglich, Löcher transportierende Polymere durch Dotieren Löcher transportierender Moleküle in Polymere wie Polystyrol und Polycarbonat zu erhalten. Geeignete Löcher transportierende Moleküle sind die bereits vorstehend genannten Moleküle.

Geeignete Elektronen transportierende Materialien für die Schicht (4) der erfindungsgemäßen OLEDs umfassen mit oxinoiden Verbindungen chelatisierte Metalle wie Tris(8-chinolinolato)aluminium (Alq₃), Verbindungen auf Phenanthrolinbasis wie 2,9-Dimethyl,4,7-diphenyl-1,10-phenanthrolin (DDPA) oder 4,7-Diphenyl-1,10-phenanthrolin (DPA) und Azolverbindungen wie 2-(4-Biphenylyl)-5-(4-t-butylphenyl)-1,3,4-oxadiazol (PBD) und 3-(4-Biphenylyl)-4-phenyl-5-(4-t-butylphenyl)-1,2,4-triazol (TAZ). Dabei kann die Schicht (4) sowohl zur Erleichterung des Elektronentransports dienen als auch als Pufferschicht oder als Sperrschicht, um ein Quenchen des Excitons an den Grenzflächen der Schichten des OLEDs zu vermeiden. Vorzugsweise verbessert die Schicht (4) die Beweglichkeit der Elektronen und reduziert ein Quenchen des Excitons.

Die Kathode (5) ist eine Elektrode, die zur Einführung von Elektronen oder negativen Ladungsträgern dient. Die Kathode kann jedes Metall oder Nichtmetall sein, das eine geringere Arbeitsfunktion aufweist als die Anode. Geeignete Materialien für die Kathode sind ausgewählt aus der Gruppe bestehend aus Alkalimetallen der Gruppe 1, zum Beispiel Li, Cs, Erdalkalimetallen der Gruppe 2, Metallen der Gruppe 12 des Periodensystems der Elemente, umfassend die Seltenerdmetalle und die Lanthanide und Aktinide. Des Weiteren können Metalle wie Aluminium, Indium, Calcium, Barium, Samarium und Magnesium sowie Kombinationen davon eingesetzt werden. Weiterhin können Lithium enthaltende organometallische Verbindungen oder LiF zwischen der organischen Schicht und der Kathode aufgebracht werden, um die Betriebsspannung (Operating Voltage) zu vermindern.

Das OLED gemäß der vorliegenden Erfindung kann zusätzlich weitere Schichten enthalten, die dem Fachmann bekannt sind. Beispielsweise kann zwischen der Schicht (2) und der Licht emittierenden Schicht (3) eine Schicht aufgebracht sein, die den Transport der positiven Ladung erleichtert und/oder die Bänderlücke der Schichten aneinander anpasst. Alternativ kann diese weitere Schicht als Schutzschicht dienen. In analoger Weise können zusätzliche Schichten zwischen der Licht emittierenden Schicht (3) und der Schicht (4) vorhanden sein, um den Transport der negativen Ladung zu erleichtern und/oder die Bänderlücke zwischen den Schichten aneinander anzupassen. Alternativ kann diese Schicht als Schutzschicht dienen.

In einer bevorzugten Ausführungsform enthält das erfindungsgemäße OLED zusätzlich zu den Schichten (1) bis (5) mindestens eine der im Folgenden genannten weiteren Schichten:
- eine Loch-Injektionsschicht zwischen der Anode (1) und der Löcher-transportierenden Schicht (2);
- eine Blockschicht für Elektronen zwischen der Löcher-transportierenden Schicht (2) und der Licht-emittierenden Schicht (3);
- eine Blockschicht für Löcher zwischen der Licht-emittierenden Schicht (3) und der Elektronen-transportierenden Schicht (4);
- eine Elektronen-Injektionsschicht zwischen der Elektronen-transportierenden Schicht (4) und der Kathode (5).

Dem Fachmann ist bekannt, wie er (zum Beispiel auf Basis von elektrochemischen Untersuchungen) geeignete Materialien auswählen muss. Geeignete Materialien für die einzelnen Schichten sind dem Fachmann bekannt und z.B. in WO 00/70655 offenbart. Des Weiteren kann jede der genannten Schichten des erfindungsgemäßen OLEDs aus zwei oder mehreren Schichten ausgebaut sein. Des Weiteren ist es möglich, dass einige oder alle der Schichten (1), (2), (3), (4) und (5) oberflächenbehandelt sind, um die Effizienz des Ladungsträgertransports zu erhöhen. Die Auswahl der Materialien für jede der genannten Schichten ist bevorzugt dadurch bestimmt, ein OLED mit einer hohen Effizienz zu erhalten.

Die Herstellung des erfindungsgemäßen OLEDs kann nach dem Fachmann bekannten Methoden erfolgen. Im Allgemeinen wird das OLED durch aufeinander folgende Dampfabscheidung (Vapor deposition) der einzelnen Schichten auf ein geeignetes Substrat hergestellt. Geeignete Substrate sind zum Beispiel Glas oder Polymerfilme. Zur Dampfabscheidung können übliche Techniken eingesetzt werden wie thermische Verdampfung, Chemical Vapor Deposition und andere. In einem alternativen Verfahren können die organischen Schichten aus Lösungen oder Dispersionen in geeigneten Lösungsmitteln beschichtet werden, wobei dem Fachmann bekannte Beschichtungstechniken angewendet werden. Zur Aufbringung der erfindungsgemäßen Polyfluoranthene ist keine Dampfabscheidung erforderlich. Die Polyfluoranthene werden im Allgemeinen entweder direkt auf der vorhergehenden Schicht polymerisiert, wobei sich der gewünschte Film (die gewünschte Schicht) enthaltend oder bestehend aus mindestens einem erfindungsgemäßen Polyfluoranthen bildet. In einer weiteren Ausführungsform erfolgt die Applikation der erfindungsgemäßen Polyfluoranthene der Formel I aus Lösung, wobei als organische Lösungsmittel beispielsweise Ether, chlorierte Kohlenwasserstoffe, zum Beispiel Methylenchlorid, und aromatische Kohlenwasserstoffe, zum Beispiel Toluol, geeignet sind. Die Applikation selber kann durch konventionelle Techniken erfolgen, zum Beispiel Spincoating, Tauchen durch filmbildendes Aufrakeln (Siebdrucktechnik), durch Auftragen mit einem Tintenstrahldrucker oder durch Stempeldruck, zum Beispiel durch PDMS, das ist Stempeldruck mittels einem Silikonkautschukstempel der photochemisch strukturiert wurde.

Im Allgemeinen haben die verschiedenen Schichten folgende Dicken: Anode (2) 500 bis 5000 Å, bevorzugt 1000 bis 2000 Å; Löcher-transportierende Schicht (3) 50 bis 1000 Å, bevorzugt 200 bis 800 Å, Licht-emittierende Schicht (4) 10 bis 2000 Å, bevorzugt 30 bis 1500 Å, Elektronen transportierende Schicht (5) 50 bis 1000 Å, bevorzugt 200 bis 800 Å, Kathode (6) 200 bis 10.000 Å, bevorzugt 300 bis 5000 Å. Die Lage der Rekombinationszone von Löchern und Elektronen in dem erfindungsgemäßen OLED und somit das Emissionsspektrum des OLED können durch die relative Dicke jeder Schicht beeinflusst werden. Das bedeutet, die Dicke der Elektronentransportschicht sollte bevorzugt so gewählt werden, dass die Elektronen/Löcher Rekombinationszone in der Licht-emittierenden Schicht liegt. Das Verhältnis der Schichtdicken der einzelnen Schichten in dem OLED ist von den eingesetzten Materialien abhängig. Die Schichtdicken von gegebenenfalls eingesetzten zusätliche Schichten sind dem Fachmann bekannt.

Durch Einsatz der erfindungsgemäßen in der Licht-emittierenden Schicht der erfindungsgemäßen OLEDs können OLEDs mit hoher Effizienz erhalten werden. Die Effizienz der erfindungsgemäßen OLEDs kann des Weiteren durch Optimierung der anderen Schichten verbessert werden. Beispielsweise können hoch effiziente Kathoden wie Ca, Ba oder LiF eingesetzt werden. Geformte Substrate und neue Löcher-transportierende Materialien, die eine Reduktion der Operationsspannung oder eine Erhöhung der Quanteneffizienz bewirken, sind ebenfalls in den erfindungsgemäßen OLEDs einsatzbar. Des Weiteren können zusätzliche Schichten in den OLEDs vorhanden sein, um die Energielevel der verschiedenen Schichten einzustellen und um Elektrolumineszenz zu erleichtern.

Die erfindungsgemäßen OLEDs können in allen Vorrichtungen eingesetzt werden, worin Elektrolumineszenz nützlich ist. Geeignete Vorrichtungen sind bevorzugt ausgewählt aus stationären und mobilen Bildschirmen. Stationäre Bildschirme sind z.B. Bildschirme von Computern, Fernsehern, Bildschirme in Druckern, Küchengeräten sowie Reklametafeln, Beleuchtungen und Hinweistafeln. Mobile Bildschirme sind z.B. Bildschirme in Handys, Laptops, Fahrzeugen sowie Zielanzeigen an Bussen und Bahnen.

Weiterhin können die erfindungsgemäßen in OLEDs mit inverser Struktur eingesetzt werden. Bevorzugt werden die erfindungsgemäßen Polyfluoranthene in diesen inversen OLEDs wiederum in der Licht-emittierenden Schicht, besonders bevorzugt als Licht-emittierende Schicht ohne weitere Zusätze, eingesetzt. Der Aufbau von inversen OLEDs und die üblicherweise darin eingesetzten Materialien sind dem Fachmann bekannt.

Somit sind die erfindungsgemäßen Polyfluoranthene der Formel I als Emitterubstanzen in organischen Leuchtdioden geeignet. Ein weiterer Gegenstand der vorliegenden Erfindung ist somit die Verwendung der erfindungsgemäßen Polyfluoranthene der Formel I als Emittersubstanzen in organischen Leuchtdioden.

Die nachfolgenden Beispiele erläutern die Erfindung zusätzlich.

### Beispiele

Die Nomenklatur der Fluoranthene im Sinne der vorliegenden Erfindung erfolgt nach dem untenstehenden Schema:

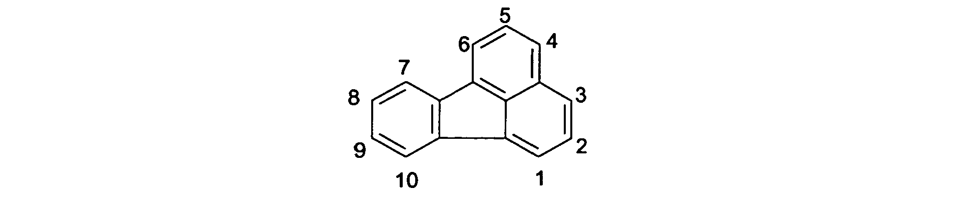

### Monomersynthesen

### 7,9-Bis-(4-bromo-phenyl)-cyclopenta[a]acenaphthylen-8-on:

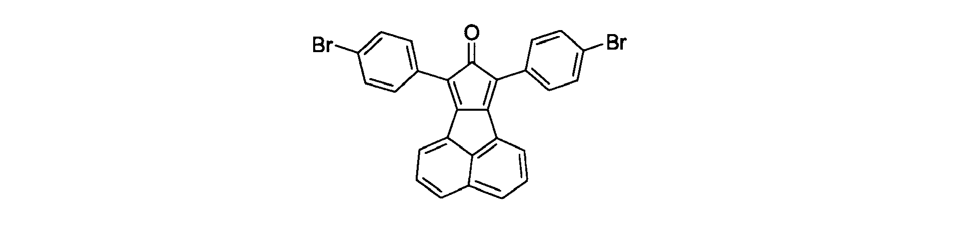

12,3 g 1,3-[Di(-4-bromophenyl)]propanon (synthetisiert nach Collman et al., J. Am. Chem. Soc. 1972, 94, 1788) und 9,06 g Acenaphthenchinon wurden in 250 ml Ethanol unter Rückfluß erhitzt. Danach gab man 2,7 g einer 20% Kaliumhydroxidlösung in Ethanol zu, wobei sich die Lösung sofort dunkelviolett führte. Nach 16 h unter Rückfluß gab man 1500 ml Ethanol zu und ließ eine weitere Stunde rühren. Die ausfallenden schwarz-grauen Kristalle (16,7 g) wurden abfiltriert und mehrmals mit Ethanol gewaschen.
Substituierte Cyclopentaacenaphthylenone sind bereits in JP 10-169992 beschrieben.

### 7,10-Bis-(4-bromo-phenyl)-8,9-diphenyl-fluoranthen:

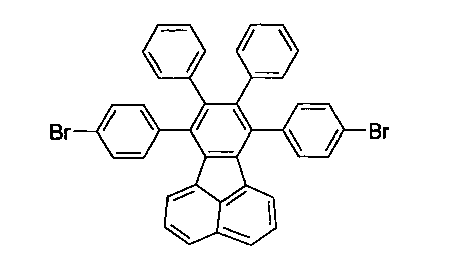

0,65 g Diphenylacetylen (darzustellen nach Herwig et al., Adv. Mater. 1996, 8, 510) und 1,9 g 7,9-Bis-(4-bromo-phenyl)-cyclopenta[a]acenaphthylen-8-on wurden in 10 g Diphenylether gelöst und 14 h unter Rückfluß erhitzt. Abdestillieren des Lösungsmittels und Chromatographie an Kieselgel (Merck Kieselgel 60, Essigester/Cyclohexan) ergaben 0,12 g eines beigen Feststoffs.

### 7,10-Bis-(4-bromo-phenyl)-8,9-bis-(4-octyl-phenyl)-fluoranthen

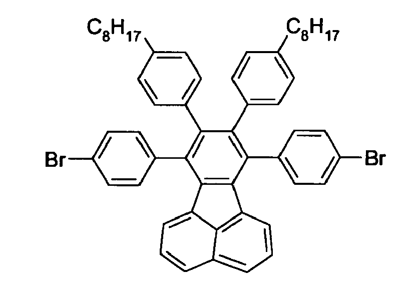

1 g 4,4'-Dioctyl-diphenylacetylen (darzustellen nach Herwig et al., Adv. Mater. 1996, 8, 510) und 1,9 g 7,9-Bis-(4-bromo-phenyl)-cyclopenta[a]acenaphthylen-8-on wurden in 10 g Diphenylether gelöst und 14 h unter Rückfluss erhitzt. Abdestillieren des Lösungsmittels und Umfällen in Methanol ergaben 0,5 g eines beigen Feststoffs.

### 7,10-Bis-(4-bromo-phenyl)-8-nonyl-9-octyl-fluoranthen:

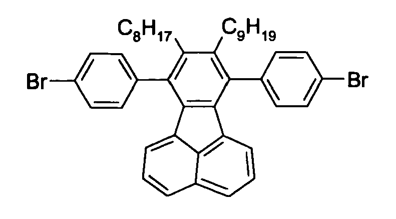

2 g 1-Nonadecin und 4 g 7,9-Bis-(4-bromo-phenyl)-cyclopenta[a]acenaphthylen-8-on wurden in 15 g Diphenylether gelöst und 16 h unter Rückfluß erhitzt. Abdestillieren des Lösungsmittels und Chromatographie an Kieselgel (Merck Kieselgel 60, Essigester/Cyclohexan) ergaben 3 g eines gelblichen Feststoffs.

### Polymersynthesen

Die Polymersynthesen wurden nach dem Fachmann bekannten Verfahren hergestellt. Polymerisationen nach Suzuki mit Palladium (0) sind beispielsweise in WO 00/22026 und WO 00/53656 beschrieben, Polymerisationen nach Yamamoto mit Nickel (0) in US 5,708,130.

### Polymerisation von 7,10-Bis-(4-bromo-phenyl)-8,9-bis-(4-octyl-phenyl)-fluoranthen

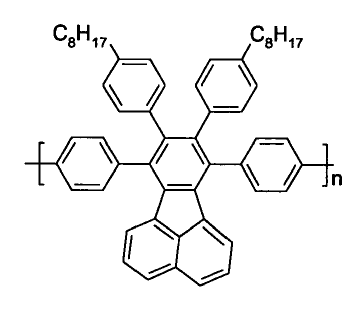

0,55 g 7,10-Bis-(4-bromo-phenyl)-8,9-bis-(4-octyl-phenyl)-fluoranthen, 0,39 g Bis (1,5-cycloocdadien)-nickel (0), 0,22 g 2,2'-Bipyridin und 0,1 g 1,5-Cyclooctadien wurden in 20 ml Dimethylformamid 3 Tage bei 80°C unter Argon erhitzt. Die Reaktionsmischung wird in einem Aceton/Methanol/Salzsäure-Gemisch gefällt, anschließend mehrmals in Methanol. Man erhält einen beige-braunen Feststoff.
M_{w} = 13100, λ_{max,em} (Toluol) = 472 nm

### Polymerisation von 7,10-Bis-(4-bromo-phenyl)-8-nonyl-9-octyl-fluoranthen

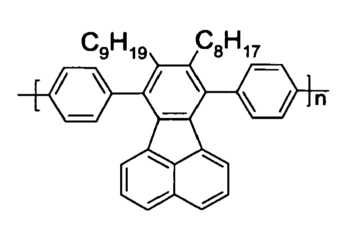

0,51 g 7,10-Bis-(4-bromo-phenyl)-8-nonyl-9-octyl-fluoranthen, 0,19 g Bis (1,5-cycloocdadien)-nickel (0), 0,11 g 2,2'-Bipyridin und 0,05 g 1,5-Cyclooctadien wurden in einem Gemisch aus 15 ml Dimethylformamid und 5 ml Toluol 3 Tage bei 80°C unter Argon erhitzt. Die Reaktionsmischung wird in einem Aceton/Methanol/Salzsäure-Gemisch gefällt, anschließend mehrmals in Methanol. Man erhält einen gelblichen Feststoff.
M_{w} = 4400, λ_{max,em} (Toluol) = 466 nm

### Polymerisation von 7,10-Bis-(4-bromo-phenyl)-8-nonyl-9-octyl-fluoranthen und Phenylen 1,3- bis(pinacolato boronat)

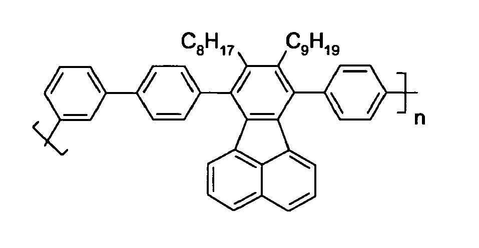

0,2 g 7,10-Bis-(4-bromo-phenyl)-8-nonyl-9-octyl-fluoranthen, 0,09 g Phenylen-1,3-bis(pinacolato boronat) (synthetisiert aus 1,3-Dibrombenzol und Bis(pinacolato boronat) nach Miyaura et al., J. Org. Chem. 1995, 60, 7508) und 0,03 g Tetrakis(triphenylphosphino)palladium (0) wurden in einem Gemisch aus 10 ml Toluol und 5 ml 40%-Kaliumcarbonatlösung 3 Tage bei 80°C unter Argon erhitzt. Die Reaktionsmischung wird anschließend mehrmals in Methanol gefällt. Man erhält einen gelblichen Feststoff.
M_{w} = 11100, λ_{max,em} (Toluol) = 468 nm

### Polymerisation von 7,10-Bis-(4-bromo-phenyl)-8-nonyl-9-octyl-fluoranthen und 2,5-Bis(hexyl)-1,4-phenylenbisboronsäure

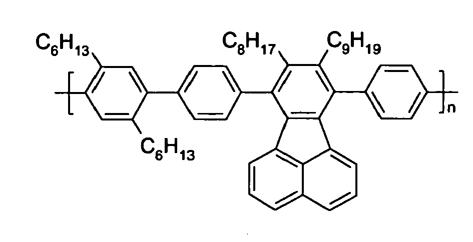

0,2 g 7,10-Bis-(4-bromo-phenyl)-8-nonyl-9-octyl-fluoranthen, 0,31 g 2,5-Bis(hexyl)-1,4-phenylenbisboronsäure (synthetisiert aus 2,5-Bis(hexyl)-1,4dibrombenzol und Bis(pinacolato boronat) nach Miyaura et al., J. Org. Chem. 1995, 60, 7508) und 0,1 g Tetrakis(triphenylphosphino)palladium (0) wurden in einem Gemisch aus 20 ml Toluol und 50 ml 40%-Kaliumcarbonatlösung 3 Tage bei 80°C unter Argon erhitzt. Die Reaktionsmischung wird anschließend mehrmals in Methanol gefällt. Man erhält einen beige-braunen Feststoff.
M_{w} = 8500, λ_{max,em} (Toluol) = 469 nm, λ_{max,em} (Film) = 469 nm

### Polymerisation von 7,10-Bis-(4-bromo-phenyl)-8-nonyl-9-octyl-fluoranthen

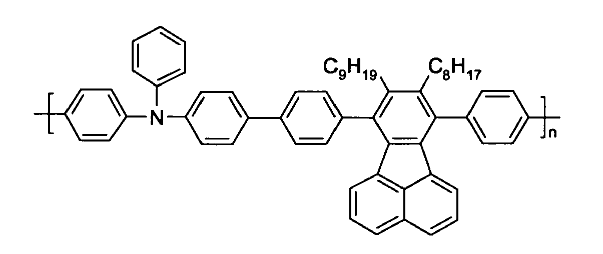

0,26 g 7,10-Bis-(4-bromo-phenyl)-8-nonyl-9-octyl-fluoranthen, 0,19 g Bis (1,5-cycloocdadien)-nickel (0), 0,14 g 2,2'-Bipyridin, 0,014 g Bis-(4-bromo-phenyl)-phenylamin und 0,06 g 1,5-Cyclooctadien wurden in einem Gemisch aus 5 ml Dimethylformamid und 5 ml Toluol 3 Tage bei 80°C unter Argon erhitzt. Die Reaktionsmischung wird in einem Aceton/Methanol/Salzsäure-Gemisch gefällt, anschließend mehrmals in Methanol. Man erhält einen gelblichen Feststoff.
M_{w} = 7300, λ_{max,em} (Toluol) = 467 nm

## Patentansprüche

1. Verfahren zur Herstellung von Polyfluoranthenen enthaltend Wiederholungseinheiten der allgemeinen Formel I 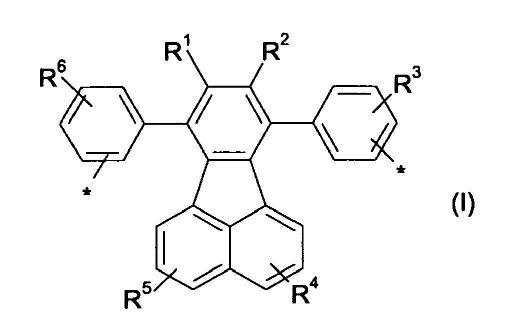 umfassend folgende Schritte:
a) Herstellung eines monomeren Fluoranthenderivats der Formel IIa 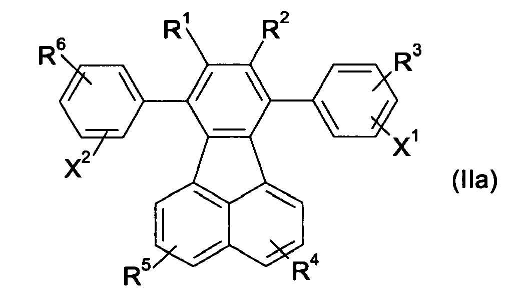 durch Reaktion einer Verbindung der Formel III 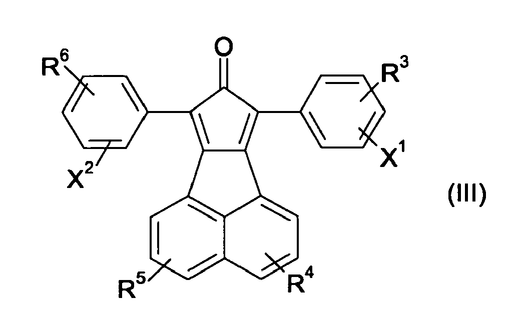 mit einer Alkinylverbindung der Formel IV 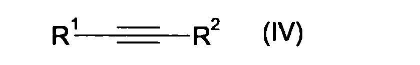 und anschließender Kohlenmonoxid-Eliminierung;
b) gegebenenfalls Umsetzung des monomeren Fluoranthenderivats der Formel IIa zu einem monomeren Fluoranthenderivat der Formel IIb 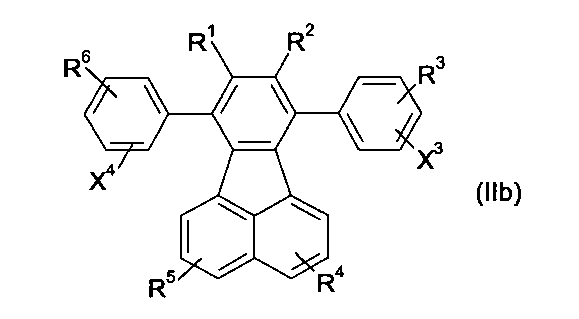
c) Polymerisation des monomeren Fluoranthenderivats der Formel IIa oder IIb, gegebenenfalls gemeinsam mit mindestens einem weiteren Comonomeren ausgewählt aus der Gruppe bestehend aus einem weiteren von dem ersten Fluoranthenderivat der Formel IIa bzw. IIb verschiedenen Fluoranthenderivat der Formel IIa oder IIb, aromatischen, kondensierten aromatischen und heteroaromatischen Verbindungen, die jeweils zwei mit den Gruppen X¹ und X² des Fluoranthenderivats der Formel IIa bzw. den Gruppen X³ und X⁴ des Fluoranthenderivats der Formel IIb polymerisierbare Gruppen X⁵ und X⁶ aufweisen;
wobei die Symbole die folgenden Bedeutungen aufweisen:
R¹, R², R³ R⁴, R⁵, R⁶ unabhängig voneinander H, ein Alkylrest, ein Alkinylrest, ein Aryloxyrest, ein aromatischer Rest, ein kondensiertes aromati- sches Ringsystem, ein heteroaromatischer Rest, -CH=CH₂, (E)- oder (Z)-CH=CH-C₆H₅, Acryloyl, Methacryloyl, Methylstyryl, -O- CH=CH₂ oder Glycidyl, 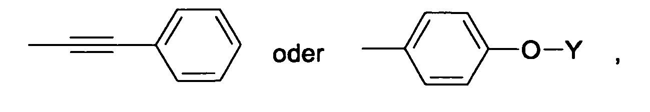 bedeuten,
wobei Y Acryloyl, Methacryloyl, ortho- oder para-Methylstyryl, - O-CH=CH₂ oder Glycidyl bedeutet;
X¹, X², X³ X⁴, X⁵, X⁶ miteinander polymerisierbare Gruppen.

2. Verfahren nach Anspruch 1, **dadurch gekennzeichnet, dass** R⁴ und R⁵ H sind.

3. Verfahren nach Anspruche 1 oder 2, **dadurch gekennzeichnet, dass** R³ und R⁶ H sind.

4. Verfahren nach einem der Ansprüche 1 bis 3, **dadurch gekennzeichnet, dass** R¹ und R² Alkylreste, bevorzugt C₃- bis C₁₀-Alkylreste, besonders bevorzugt C₅- bis C₉-Alkylreste, die insbesondere linear sind, bedeuten.

5. Verfahren nach einem der Ansprüche 1 bis 4, **dadurch gekennzeichnet, dass** die Polymerisation in Schritt c) in Gegenwart von Nickel- oder Palladiumverbindungen durchgeführt wird.

6. Verfahren nach Anspruch 5, **dadurch gekennzeichnet, dass** X¹, X² bzw. X³ und X⁴, X⁵ und X⁶ die folgenden Bedeutungen aufweisen:
X¹ und X² jeweils Halogen, ausgewählt aus F, Cl, Br und I; bzw.
X³ und X⁴ Halogen, ausgewählt aus F, Cl, Br und I, verestertes Sulfonat oder einen Bor-haltigen Rest der Formel -B(O-[C(R⁷)₂]ₙ-O und
X⁵ und X⁶ Halogen, ausgewählt aus F, Cl, Br und I, verestertes Sulfonat oder einen Bot-haltigen Rest der Formel -B(O-[C(R⁷)₂]ₙ-O;
R⁷ gleich oder verschieden unabhängig voneinader H oder C₁-C₂₀-Alkyl;
n eine ganze Zahl von 2 bis 10;
mit der Maßgabe, dass
die Reste X¹ und X² bzw. X³ und X⁴ sowie X⁵ und X⁶ so ausgewählt werden, dass das molare Verhältnis von Halogen und verestertem Sulfonat zu Bor-haltigem Rest 0,8 zu 1 bis 2,1 zu 1 beträgt,
oder
die Reste in dem monomeren Fluoranthenderivat jeweils Halogen bedeuten und diese gegebenenfalls gemeinsam mit weiteren Comonomeren umgesetzt werden, worin die Reste X⁵ und X⁶ ebenfalls jeweils Halogen bedeuten.

7. Verfahren nach Anspruch 5 oder 6, **dadurch gekennzeichnet, dass** das mindestens eine weitere Comonomere ausgewählt ist aus der Gruppe bestehend aus Phenylbisboronsäuren, Phenylenbisboronsäureestern, den alkyl- oder alkoxysubstituierten Derivaten der Phenylenbisboronsäuren bzw. Phenylenbisboronsäureester; dihalogensubstituierten Benzolen, alkyl- oder alkoxysubstituierten Derivaten der dihalogensubstituierten Benzole; Anthracenbisboronsäuren, Anthracenbisboronsäureestern, Dihaloanthracen, dihalogensubstituierten Triarylaminen, den Bisboronsäuren von dihalogensubstituierten Triarylaminen, den Estern der Bisboronsäuren von dihalogensubstituierten Triarylaminen, den alkyl- oder alkoxysubstituierten Derivaten der dihalogensubstituierten Triarylamine, ihrer Bisboronsäuren, ihrer Bisboronsäureester, dihalogensubstituierten Naphthalinen, den Bisboronsäuren der halogensubstituierten Naphthaline, den Bisboronsäureestern der dihalogensubsituierten Naphthaline, ihren alkyl- oder alkoxysubstituierten Derivaten; dihalogensubstituierten Fluorenen, den Bisboronsäuren der dihalogensubstituierten Fluorene, den Estern der Bisboronsäuren der dihalogensubstituierten Fluorene, ihren alkyl- oder alkoxysubstituierten Derivaten; dihalogensubstituierten Carbazolen, den Bisboronsäuren der dihalogensubstituierten Carbazole, den Bisboronsäureestern der dihalogensubstituierten Carbazole, ihren alkyl- oder alkoxysubstituierten Derivaten; dihalogensubstituierten Dibenzofuranen, den Bisboronsäuren der dihalogensubstituierten Dibenzofurane, den Bisboronsäureestern der dihalogensubstituierten Dibenzofurane, ihren alkyl- oder alkoxysubstituierten Derivaten; dihalogensubstituierten Pyrenen, den Bisboronsäuren der dihalogensubstituierten Pyrene, den Bisboronsäureestern der dihalogensubstituierten Pyrene, ihren alkyl- oder alkoxysubstituierten Derivaten, dihalogensubstituierten Phenanthrenen, den Bisboronsäuren der dihalogensubstituierten Phenanthrene, den Estern der Bisboronsäuren der dihalogensubstituierten Phenanthrene und deren alkyl- oder alkoxysubstituierten Derivaten.

8. Verfahren nach Anspruch 7, **dadurch gekennzeichnet, dass** das mindestens eine weitere Comonomere ausgewählt ist aus der Gruppe bestehend aus Phenylenbisboronsäuren, Phenylenbisboronsäureestern und dihalogensubstituierten Triarylaminen.

9. Polyfluoranthene herstellbar nach einem Verfahren gemäß einem der Ansprüche 1 bis 8.

10. Filme enthaltend oder bestehend aus mindestens einem Polyfluoranthen gemäß Anspruch 9.

11. Organische Leuchtdioden enthaltend mindestens ein Polyfluoranthen gemäß Anspruch 9.

12. Licht-emittierende Schicht enthaltend oder bestehend aus mindestens einem Polyfluoranthen gemäß Anspruch 9.

13. Organische Leuchtdiode enthaltend eine Licht-emittierende Schicht gemäß Anspruch 12.

14. Vorrichtung ausgewählt aus der Gruppe bestehend aus stationären Bildschirmen wie Bildschirmen von Computern, Fernsehern, Bildschirmen in Druckern, Küchengeräten sowie Reklametafeln, Beleuchtungen, Hinweistafeln und mobilen Bildschirmen wie Bildschirmen in Handys, Laptops, Fahrzeugen sowie Zielanzeigen an Bussen und Bahnen, enthaltend ein OLED gemäß Anspruch 11 oder 13.

15. Verwendung von Polyfluoranthenen gemäß Anspruch 9 als Emittersubstanzen in organischen Leuchtdioden.

## Claims

1. A process for preparing polyfluoranthenes comprising repeating units of the general formula 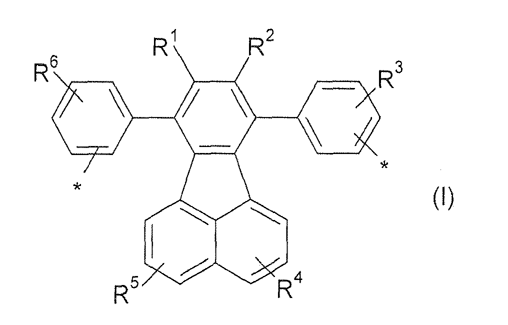 which comprises the following steps:
a) Preparation of a monomeric fluoranthene derivative of the formula IIa 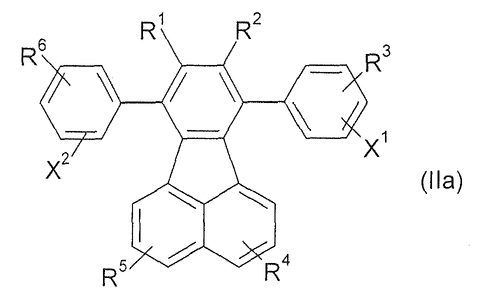 by reaction of a compound of the formula III 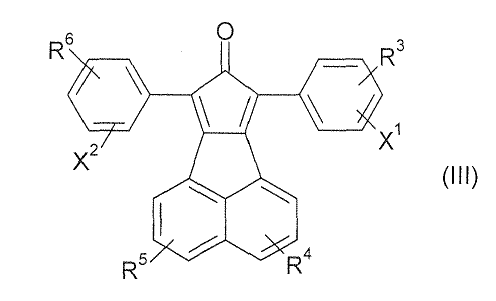 with an alkynyl compound of the formula IV 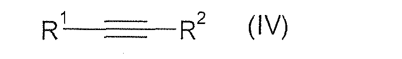 and subsequent carbon monoxide elimination;
b) if appropriate, reaction of the monomeric fluoranthene derivative of the formula IIa to form a monomeric fluoranthene derivative of the formula IIb 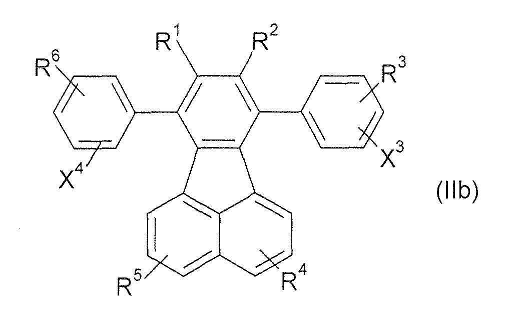
c) Polymerization of the monomeric fluoranthene derivative of the formula IIa or IIb, if appropriate together with at least one further comonomer selected from the group consisting of further fluoranthene derivatives of the formula IIa or IIb which are different from the first fluoranthene derivative of the formula IIa or IIb, aromatic, fused aromatic and heteroaromatic compounds which each have two groups X⁵ and X⁶ which can be polymerized with the groups X¹ und X² of the fluoranthene derivative of the formula IIa or the groups X³ and X⁴ of the fluoranthene derivative of the formula IIb;
where the symbols have the following meanings:
R¹,R²,R³ R⁴, R⁵, R⁶ are each, independently of one another, H, an alkyl radical, an alkynyl radical, an aryloxy radical, an aromatic radical, a fused aromatic ring system, a heteroaromatic radical, -CH=CH₂, trans- or cis-CH=CH-C₆H₅, acryloyl, methacryloyl, methylstyryl, -O-CH=CH₂ or glycidyl, 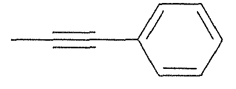 or 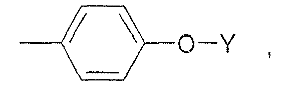 where Y is acryloyl, methacryloyl, ortho- or para- methylstyryl, -O-CH=CH₂ or glycidyl;
X¹, X²,X³ X⁴, X⁵, X⁶ are groups which can be polymerized with one another.

2. The process according to claim 1, wherein R⁴ and R⁵ are each H.

3. The process according to claim 1 or 2, wherein R³ and R⁶ are each H.

4. The process according to any of claims 1 to 3, wherein R¹ and R² are alkyl radicals, preferably C₃-C₁₀-alkyl radicals, particularly preferably C₅-C₉-alkyl radicals, which are very particularly preferably linear.

5. The process according to any of claims 1 to 4, wherein the polymerization in step c) is carried out in the presence of nickel or palladium compounds.

6. The process according to claim 5, wherein X¹, X² or X³ and X⁴, X⁵ and X⁶ have the following meanings:
X¹ and X² are each halogen selected from among F, Cl, Br and l; or
X³ and X⁴ are each halogen selected from among F, Cl, Br and I, esterified sulfonate or a boron-comprising radical of the formula -B(O-[C(R⁷)₂]ₙ-O and
X⁵ and X⁶ are each halogen selected from among F, Cl, Br and I, esterified sulfonate or a boron-comprising radical of the formula -B(O-[C(R⁷)₂]ₙ-O;
the radicals R⁷ are identical or different and are each, independently of one another, H or C₁-C₂₀-alkyl;
n is an integer from 2 to 10;
with the proviso that
the radicals X¹ and X² or X³ and X⁴ and also X⁵ and X⁶ are selected so that the molar ratio of halogen and esterified sulfonate to boron-comprising radical is from 0.8:1 to 2.1:1,
or
the radicals in the monomeric fluoranthene derivative are each halogen and these are reacted, if appropriate together with further comonomers in which the radicals X⁵ and X⁶ are likewise each halogen.

7. The process according to claim 5 or 6, wherein the at least one further comonomer is selected from the group consisting of phenylenebisboronic acids, phenylenebisboronic esters, the alkyl- or alkoxy-substituted derivatives of the phenylenebisboronic acids or phenylenebisboronic esters; dihalo-substituted benzenes, alkyl- or alkoxy-substituted derivatives of the dihalo-substituted benzenes; anthracenebisboronic acids, anthracenebisboronic esters, dihaloanthracene, dihalo-substituted triarylamines, the bisboronic acids of dihalo-substituted triarylamines, the esters of the bisboronic acids of dihalo-substituted triarylamines, the alkyl- or alkoxy-substituted derivatives of the dihalo-substituted triarylamines, their bisboronic acids, their bisboronic esters, dihalo-substituted naphthalenes, the bisboronic acids of the halo-substituted naphthalenes, the bisboronic esters of the dihalo-substituted naphthalenes, their alkyl- or alkoxy-substituted derivatives; dihalo-substituted fluorenes, the bisboronic acids of the dihalo-substituted fluorenes, the esters of the bisboronic acids of the dihalo-substituted fluorenes, their alkyl- or alkoxy-substituted derivatives; dihalo-substituted carbazoles, the bisboronic acids of the dihalo-substifiuted carbazoles, the bisboronic esters of the dihalo-substituted carbazoles, their alkyl- or alkoxy-substituted derivatives; dihalo-substituted dibenzofurans, the bisboronic acids of the dihalo-substituted dibenzofurans, the bisboronic esters of the dihalo-substituted dibenzofurans, their alkyl- or alkoxy-substituted derivatives; dihalo-substituted pyrenes, the bisboronic acids of the dihalo-substituted pyrenes, the bisboronic esters of the dihalo-substituted pyrenes, their alkyl- or alkoxy-substituted derivatives, dihalo-substituted phenanthrenes, the bisboronic acids of the dihalo-substituted phenanthrenes, the esters of the bisboronic acids of the dihalo-substituted phenanthrenes and their alkyl- or alkoxy-substituted derivatives.

8. The process according to claim 7, wherein the at least one further comonomer is selected from the group consisting of phenylenebisboronic acids, phenylenebisboronic esters and dihalo-substituted triarylamines.

9. A polyfluoranthene which can be prepared by a process according to any of claims 1 to 8.

10. A film comprising or consisting of at least one polyfluoranthene according to claim 9.

11. An organic light-emitting diode comprising at least one polyfluoranthene according to claim 9.

12. A light-emitting layer comprising or consisting of at least one polyfluoranthene according to claim 9.

13. An organic light-emitting diode comprising a light-emitting layer according to claim 12.

14. A device selected from the group consisting of stationary VDUs such as VDUs of computers, televisions, VDUs in printers, kitchen appliances and also advertising signs, lighting, information signs and mobile VDUs such as VDUs in mobile telephones, laptops, vehicles and also destination displays on buses and trains which comprises an OLED according to claim 11 or 13.

15. The use of polyfluoranthenes according to claim 9 as emitter substances in organic light-emitting diodes.

## Revendications

1. Procédé pour la préparation de polyfluoranthènes contenant des motifs répétitifs de formule générale I 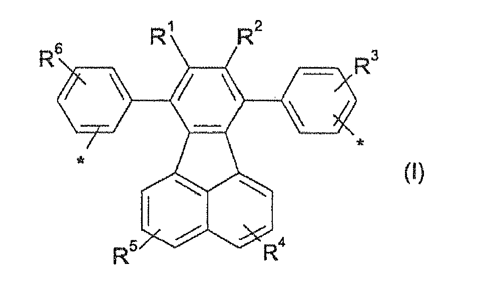 comprenant les étapes suivantes:
a) préparation d'un dérivé de fluoranthène monomère de formule IIa 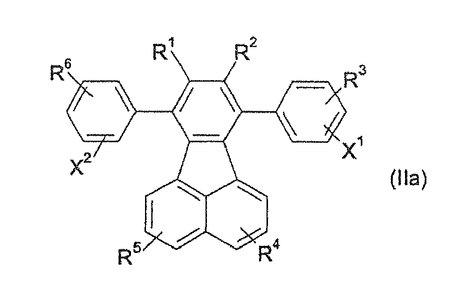 par réaction d'un composé de formule III 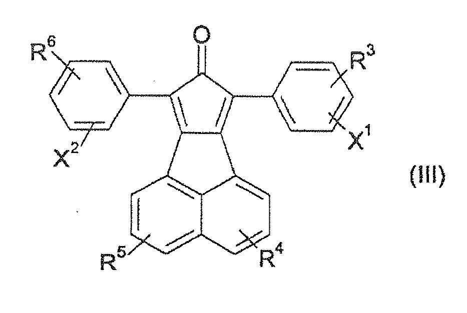 avec un composé alcynyle de formule IV 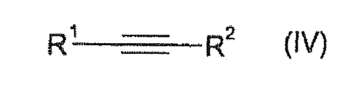 et élimination subséquente de monoxyde de carbone ;
b) éventuellement conversion du dérivé de fluoranthène monomère de formule IIa en un dérivé de fluoranthène monomère de formule IIb 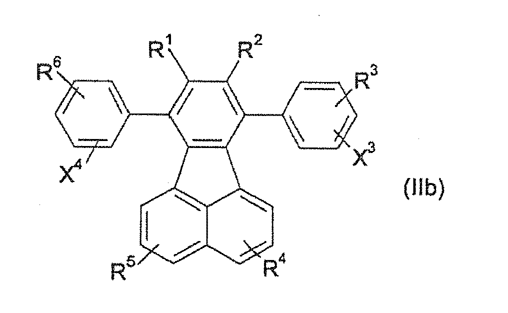
c) polymérisation du dérivé de fluoranthène monomère de formule IIa ou IIb, éventuellement conjointement avec au moins un autre comonomère choisi dans le groupe constitué par un autre dérivé de fluoranthène de formule IIa ou IIb, différent du premier dérivé de fluoranthène de formule IIa ou IIb, des composés aromatiques, aromatiques condensés et hétéroaromatiques, qui comportent chacun deux groupes X⁵ et X⁶ polymérisables avec les groupes X¹ et X² du dérivé de fluoranthène de formule IIa ou les groupes X³ et X⁴ du dérivé de fluoranthène de formule IIb ;
les symboles ayant les significations suivantes:
R¹, R² , R³, R⁴, R⁵, R⁶ représentent, indépendamment les uns des autres, H, un radical alkyle, un radical alcynyle, un radical aryloxy, un radical aromatique, un système cyclique aromatique condensé, un radical hétéroaromatique, -CH=CH₂, (E)- ou (Z)-CH=CH-C₆H₅, un groupe acryloyle, méthacryloyle, méthyl- styryle, -O-CH=CH₂ ou glycidyle, 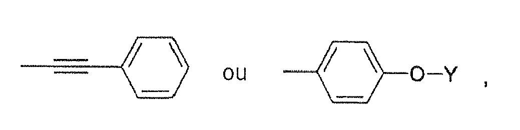 Y représentant un groupe acryloyle, méthacryloyle, ortho- ou para-méthylstyryle, -O-CH=CH₂ ou glycidyle ;
X¹, X², X³, X⁴, X⁵, X⁶ représentent des groupes polymérisables entre eux.

2. Procédé selon la revendication 1, **caractérisé en ce que** R⁴ et R⁵ sont des atomes d'hydrogène.

3. Procédé selon la revendication 1 ou 2, **caractérisé en ce que** R³ et R⁶ sont des atomes d'hydrogène.

4. Procédé selon l'une quelconque des revendications 1 à 3, **caractérisé en ce que** R¹ et R² représentent des radicaux alkyle, de préférence des radicaux alkyle en C₃-C₁₀, de façon particulièrement préférée des radicaux alkyle en C₅-C₉, qui sont en particulier linéaires.

5. Procédé selon l'une quelconque des revendications 1 à 4, **caractérisé en ce que** la polymérisation dans l'étape c) est effectuée en présence de composés contenant du nickel ou du palladium.

6. Procédé selon la revendication 5, **caractérisé en ce que** X¹, X² ou X³ et X⁴, X⁵ et X⁶ ont les significations suivantes:
X¹ et X² représentent chacun un atome d'halogène, choisi parmi F, Cl, Br et I ; ou
X³ et X⁴ représentent un atome d'halogène, choisi parmi F, Cl, Br et I, un sulfonate estérifié ou un radical contenant du bore, de formule -B (O)-[C(R⁷)₂]ₙ-O et
X⁵ et X⁶ représentent un atome d'halogène, choisi parmi F, Cl, Br et I, un sulfonate estérifié ou un radical contenant du bore, de formule -B(O)-[C(R⁷)₂]ₙ-O;
R⁷ sont identiques ou différents et représentent, indépendamment les uns des autres, H ou un groupe alkyle en C₁-C₂₀ ;
n représente un nombre entier allant de 2 à 10 ;
étant entendu que
les radicaux X¹ et X² ou X³ et X⁴ ainsi que X⁵ et X⁶ sont choisis de manière que le rapport molaire de l'halogène et du sulfonate estérifié au radical contenant du bore ait une valeur de 0,8:1 à 2,1:1,
ou
les radicaux dans le dérivé de fluoranthène monomère représentent chacun un atome d'halogène et éventuellement on les fait réagir conjointement avec d'autres comonomères dans lesquels les radicaux X⁵ et X⁶ représentent également chacun un atome d'halogène.

7. Procédé selon la revendication 5 ou 6, **caractérisé en ce que** ledit au moins un autre comonomère est choisi dans le groupe constitué par les acides phénylènebisboroniques, les esters d'acides phénylènebisboroniques, les dérivés à substitution alkyle ou alcoxy des acides phénylènebisboroniques ou des esters d'acides phénylènebisboroniques ; les benzènes disubstitués par halogène, des dérivés à substitution alkyle ou alcoxy des benzènes disubstitués par halogène ; les acides anthracènebisboroniques, les esters d'acides anthracènebisboroniques, un dihalogénoanthacène, les triarylamines disubstituées par halogène, les acides bisboroniques de triarylamines disubstituées par halogène, les esters des acides bisboroniques de triarylamines disubstituées par halogène, les dérivés à substitution alkyle ou alcoxy des triarylamines disubstituées par halogène, leurs acides bisboroniques, leurs esters d'acides bisboroniques, les naphtalènes disubstitués par halogène, les acides bisboroniques des naphtalènes disubstitués par halogène, les esters d'acides bisboroniques des naphtalènes disubstitués par halogène, leurs dérivés à substitution alkyle ou alcoxy ; les fluorènes disubstitués par halogène, les acides bisboroniques des fluorènes disubstitués par halogène, les esters d'acides bisboroniques des fluorènes disusubstitués par halogène, leurs dérivés à substitution alkyle ou alcoxy ; les carbazoles disubstitués par halogène, les acides bisboroniques des carbazoles disubstitués par halogène, les esters d'acides bisboroniques des carbazoles disusubstitués par halogène, leurs dérivés à substitution alkyle ou alcoxy ; les dibenzofurannes disubstitués par halogène, les acides bisboroniques des dibenzofurannes disubstitués par halogène, les esters d'acides bisboroniques des dibenzofurannes disusubstitués par halogène, leurs dérivés à substitution alkyle ou alcoxy ; les pyrènes disubstitués par halogène, les acides bisboroniques des pyrènes disubstitués par halogène, les esters d'acides bisboroniques des pyrènes disusubstitués par halogène, leurs dérivés à substitution alkyle ou alcoxy, les phénanthrènes disubstitués par halogène, les acides bisboroniques des phénanthrènes disubstitués par halogène, les esters d'acides bisboroniques des phénanthrènes disusubstitués par halogène et leurs dérivés à substitution alkyle ou alcoxy.

8. Procédé selon la revendication 7, **caractérisé en ce qu'**au moins un autre comonomère est choisi dans le groupe constitué par les acides phénylènebisboroniques, les esters d'acides phénylènebisboroniques et les triarylamines disubstituées par halogène.

9. Polyfluoranthènes pouvant être préparés conformément à un procédé selon l'une quelconque des revendications 1 à 8.

10. Films contenant ou consistant en au moins un polyfluoranthène selon la revendication 9.

11. Diodes électroluminescentes organiques contenant au moins un polyfluoranthène selon la revendication 9.

12. Couche luminescente contenant ou consistant en au moins un polyfluoranthène selon la revendication 9.

13. Diode électroluminescente organique contenant une couche luminescente selon la revendication 12.

14. Dispositif choisi dans le groupe constitué par des écrans fixes tels que des écrans d'ordinateurs, des téléviseurs, des écrans dans des imprimantes, des appareils de cuisine ainsi que des tableaux publicitaires, des éclairages, des tableaux indicateurs et des écrans mobiles tels que des écrans dans des téléphones portables, des ordinateurs portables, des véhicules ainsi que des afficheurs de destination dans des autobus et des trains, contenant une OLED selon la revendication 11 ou 13.

15. Utilisation de polyfluoranthènes selon la revendication 9, en tant que substances émettrices dans des diodes électroluminescentes organiques.
